# FASCICULE DE BREVET EUROPEEN

(11) **EP 2 969 902 B1**
(45) Date de publication et mention de la délivrance du brevet: **24.08.2022**
(21) Numéro de dépôt: 14720642.9
(22) Date de dépôt: 13.03.2014
(51) Int. Cl.: B67D 7/02, A61M 5/148, A61J 1/10

(54) **RÉCEPTION, VIDAGE ET TRANSFERT SOUS PRESSION D'UNE GRANDE QUANTITÉ DE FLUIDE BIOPHARMACEUTIQUE EN VUE D'UN TRAITEMENT ULTÉRIEUR**
EMPFANG, ENTWÄSSERUNG UND ÜBERTRAGUNG EINER GROSSEN MENGE EINER BIOPHARMAZEUTISCHEN FLÜSSIGKEIT UNTER DRUCK ZUR WEITERBEHANDLUNG
RECEPTION, DRAINING AND TRANSFER OF A HIGH QUANTITY OF BIOPHARMACEUTICAL FLUID UNDER PRESSURE WITH A VIEW TO SUBSEQUENT TREATMENT

(30) Priorité: 13.03.2013 FR 1352246
(43) Date de publication de la demande: 20.01.2016
(73) Titulaire: SARTORIUS STEDIM FMT SAS, 13400 Aubagne (FR)
(72) Inventeur: GENTILE, Cédric, 13090 Aix en Provence (FR); GIOVANI, Maurizio, 53012 Chiusdino (SI) (IT); TRUZZI, Paolo, 50021 Barberino Val D'Elsa (FI) (IT); AICARDI, Laurent, 13780 Cuges Les Pins (FR); SVETE, Sébastien, 13400 Aubagne (FR)
(74) Mandataire: Novagraaf International SA
(86) Numéro de dépôt international: PCT/FR2014/050583
(87) Numéro de publication internationale: WO 2014/140494

(56) Documents cités:
- EP-A1- 1 923 082
- WO-A2-2006/122179
- FR-A1- 2 682 602
- FR-A1- 2 850 582
- FR-A1- 2 956 092
- US-A- 3 838 794
- US-A- 5 163 909
- US-A- 5 399 166
- US-A- 5 799 830

## Description

L'invention concerne la réception puis le vidage et transfert sous pression contrôlée d'une grande quantité de fluide biopharmaceutique en vue d'un traitement ultérieur.

Elle a pour objet un tel dispositif de réception et vidage apte et spécialement destiné à cette fin, un système pour la réception et le transfert sous pression contrôlée d'un fluide biopharmaceutique comprenant un tel dispositif, et un procédé pour la réception et le transfert sous pression contrôlée d'un fluide biopharmaceutique, dans lequel on met en œuvre un tel système.

Dans le processus d'élaboration d'un fluide biopharmaceutique par un laboratoire d'élaboration de produits pharmaceutiques, il est connu de réceptionner une grande quantité ce fluide biopharmaceutique dans un récipient stérile adapté à cet effet, dont la contenance est de l'ordre de typiquement 10 litres à 20 litres, puis, lorsque cela est souhaité, de vider ce récipient du fluide biopharmaceutique et de le transférer en vue d'un traitement ultérieur tel que, typiquement, une filtration ou analogue, suivie d'une formulation finale ou du remplissage de conteneurs de plus petite contenance. Toutes ces opérations doivent être réalisées de façon stérile et, pour des impératifs industriels, assez rapidement. En outre, si une étape de filtration ou analogue est envisagée, le fluide biopharmaceutique doit, en amont, être sous une pression suffisante compte tenu de la perte de charge dans le filtre.

Le domaine de l'invention est celui, spécifique, de l'élaboration d'un fluide biopharmaceutique par un laboratoire d'élaboration de produits pharmaceutiques, où la réception puis le vidage et transfert portent sur une grande quantité de fluide biopharmaceutique, au moins égale à de l'ordre de 10 l, en vue d'un traitement ultérieur tel que filtration, formulation finale, remplissage de conteneurs de plus petite contenance.

Pour réaliser les opérations exposées précédemment, il est connu de mettre en œuvre un système pour la réception et le transfert sous pression contrôlée d'un fluide biopharmaceutique, comprenant un dispositif de réception puis de vidage sous pression contrôlée d'un fluide biopharmaceutique, un moyen apte et destiné à délivrer un gaz de compression sous pression ayant une ligne d'injection de gaz de compression sous pression, et un moyen de contrôle et de commande de la pression du gaz de compression sous pression dans la ligne d'injection. Les quantités de fluide biopharmaceutique réceptionnées puis transférées peuvent être typiquement de l'ordre d'une ou de plusieurs dizaines de litres.

Dans une première réalisation connue, le dispositif comprend un réceptacle rigide en acier inoxydable, pourvu d'un couvercle amovible, formant une enceinte intérieure apte et destinée à recevoir le fluide biopharmaceutique, une entrée d'emplissage de l'enceinte avec le fluide biopharmaceutique située en partie supérieure, une sortie de vidage de l'enceinte du fluide biopharmaceutique située en partie inférieure et une entrée de gaz de vidage sous pression. Un tel dispositif peut être utilisé à plusieurs reprises moyennant un nettoyage sérieux. Un tel dispositif présente comme inconvénients, notamment, que l'opération de nettoyage en vue du réemploi est longue, coûteuse et nécessite un très grand soin et que le gaz de vidage est en contact avec le fluide biopharmaceutique ce qui n'est pas souhaitable s'agissant d'un processus stérile.

Dans une seconde réalisation connue, illustrée par le document US 5 799 830, le dispositif comprend, en premier lieu, une poche intérieure en matière plastique, souple et étanche, ayant une enceinte intérieure apte et destinée à recevoir du fluide biopharmaceutique pourvue d'un orifice d'emplissage avec le fluide biopharmaceutique et d'un orifice de vidage du fluide biopharmaceutique et, associés à ces orifices, un tube d'emplissage ayant une entrée d'emplissage de l'enceinte avec le fluide biopharmaceutique et un tube de vidage ayant une sortie de vidage de l'enceinte du fluide biopharmaceutique. Le dispositif comprend, en second lieu, un conteneur extérieur rigide en acier inoxydable dans lequel est placée l'enceinte intérieure de sorte qu'une chambre de compression soit ménagée entre le conteneur extérieur et l'enceinte intérieure et que le tube d'emplissage et le tube vidage débouchent, par leur entrée et leur sortie respectivement, à l'extérieur du conteneur extérieur. Le conteneur extérieur est pourvu d'une entrée d'injection de gaz de vidage sous pression dans la chambre de compression. Avec un tel dispositif, le conteneur extérieur peut être utilisé à plusieurs reprises, comme pour la première réalisation décrite, avec les inconvénients inhérents. D'autre part, un tel dispositif est complexe puisque le conteneur extérieur doit comporter une trappe pour la mise en place et l'enlèvement de la poche intérieure qui doit pouvoir être ouverte et fermée de façon étanche, et des systèmes d'emplissage et de vidage associés de façon communicante avec la poche intérieure et associés de façon fixe et étanche avec le conteneur extérieur. Cette trappe et ces systèmes d'emplissage et de vidage rendent le nettoyage du conteneur extérieur d'autant plus complexe. Enfin, le tube de vidage traverse la poche intérieure de part en part de sorte que lorsque celle-ci est le plus possible comprimée, il reste à l'intérieur un volume résiduel dont le vidage est impossible ou difficile.

Par conséquent, il existe, dans le domaine spécifique de l'invention, le besoin de pouvoir réceptionner puis vider et transférer sous une pression suffisante et contrôlée une grande quantité de fluide biopharmaceutique, au moins égale à de l'ordre de 10 l.

Par ailleurs, il est connu de l'état de la technique antérieure des dispositifs et procédés de perfusion d'un liquide dans le corps humain, qui portent sur des quantités de liquide typiquement inférieures à 3 litres. Les dispositifs de perfusion par simple gravité sont bien connus. Il existe également des dispositifs dans lesquels la vitesse d'écoulement est régulée en exerçant sur la poche contenant le liquide à perfuser une pression de compaction au moyen d'une enceinte emplie d'un gaz comme décrits par exemple dans les documents US 3 838 794, FR-A-2 682 602, FR 2 850 582, US 5 163 909, US 5 399 166 et EP 1 923 082.

De tels dispositifs et procédés ne font pas partie du domaine spécifique de l'invention. En effet, avec ces dispositifs et procédés, contrairement au domaine de l'invention, le fluide en cause est un liquide de perfusion ou un liquide parentéral ou analogue, seulement utilisé pour être délivré à un patient, typiquement dans un centre de soins. De plus, contrairement au domaine de l'invention, la réception puis le vidage portent sur de petits quantités, au plus égales à 3 litres et le plus souvent même, nettement moins. Enfin, par comparaison au domaine de l'invention, les pressions appliquées sont beaucoup plus faibles, et les exigences quant à la délivrance des contenus une fois vidés sont différentes.

Ainsi, pour s'en tenir au seuil document US 3 838 794, celui-ci fait grand cas du colmatage du dispositif par contact des parois, problème qui peut en effet se poser avec des dispositifs de petites contenances et de grande flexibilité, mais qui est étranger au domaine de l'invention où les contenances sont beaucoup plus grandes et les dispositifs beaucoup moins flexibles.

Ci-après, un exposé de l'invention telle que caractérisée dans les revendications.

Selon un premier aspect, dans lequel l'on part comme état de la technique de la seconde réalisation connue précédemment exposée, l'invention a pour objet un dispositif de réception puis de vidage sous pression contrôlée par un laboratoire d'élaboration de produits pharmaceutiques d'un fluide biopharmaceutique en grande quantité, au moins égale à de l'ordre de 10 l, en vue d'un traitement ultérieur tel que filtration, formulation finale, remplissage de conteneurs de plus petite contenance, du type comprenant :
▪ une poche intérieure en matière plastique, souple et étanche, ayant une enceinte intérieure apte et destinée à recevoir une quantité au moins égale à de l'ordre de 10 l de fluide biopharmaceutique et pourvue d'un orifice d'emplissage avec le fluide biopharmaceutique et d'un orifice de vidage du fluide biopharmaceutique et, associés de façon étanche à l'orifice d'emplissage et à l'orifice de vidage, un tube d'emplissage ayant une entrée d'emplissage de l'enceinte avec le fluide biopharmaceutique, apte à être associé à une ligne d'emplissage du fluide biopharmaceutique, et un tube de vidage ayant une sortie de vidage de l'enceinte du fluide biopharmaceutique, apte à être associé à une ligne de vidage du fluide biopharmaceutique,
▪ un conteneur extérieur dans lequel est placée la poche intérieure, une chambre de compression étant ménagée entre le conteneur extérieur et la poche intérieure dont l'entrée d'emplissage et la sortie de vidage sont situées à l'extérieur du conteneur extérieur, un orifice d'injection de gaz de vidage sous pression dans la chambre de compression étant pourvu sur ledit conteneur extérieur,
▪ des traversées étanches du conteneur extérieur par le tube d'emplissage et le tube de vidage,
▪ les capacités de déformation respectivement de la poche intérieure et du conteneur extérieur étant choisies de sorte que lorsque l'on injecte le gaz de vidage sous pression dans la chambre de compression, la poche intérieure soit comprimé et que le fluide biopharmaceutique qui s'y trouve en soit vidé sous pression par la sortie de vidage.

Le dispositif selon ce premier aspect est tel que :
▪ le conteneur extérieur comprend un réceptacle extérieur en matière plastique, étanche, formant une enceinte extérieure dans laquelle est placée la poche intérieure, ménageant la chambre de compression, et comportant l'orifice d'injection de gaz de vidage sous pression,
▪ le tube d'emplissage et le tube de vidage traversent le réceptacle extérieur par des liaisons fixes à demeure (35), l'entrée d'emplissage et la sortie de vidage étant situées à l'extérieur du réceptacle extérieur,
▪ il comprend également un moyen intégré de purge du gaz emplissant la ligne d'emplissage avant emplissage avec le fluide biopharmaceutique de sorte que ce gaz ne pénètre pas dans la poche intérieure,
▪ le réceptacle extérieur et la poche intérieure forment un tout cohérent à usage unique.

Selon une réalisation, le dispositif de réception puis de vidage sous pression contrôlée d'un fluide biopharmaceutique comprend
▪ une poche intérieure en matière plastique, souple et étanche, ayant une enceinte intérieure apte et destinée à recevoir du fluide biopharmaceutique et pourvue d'un orifice d'emplissage du fluide biopharmaceutique et d'un orifice de vidage du fluide biopharmaceutique et, associés de façon étanche à l'orifice d'emplissage et à l'orifice de vidage, un tube d'emplissage ayant une entrée d'emplissage de l'enceinte avec le fluide biopharmaceutique, apte à être associé à une ligne d'emplissage du fluide biopharmaceutique, et un tube de vidage ayant une sortie de vidage de l'enceinte du fluide biopharmaceutique, apte à être associé à une ligne de vidage du fluide biopharmaceutique,
▪ un conteneur extérieur comprenant un réceptacle extérieur en matière plastique, étanche, formant une enceinte extérieure dans laquelle est placée la poche intérieure dont l'entrée d'emplissage et la sortie de vidage sont situées à l'extérieur du conteneur extérieur, ménageant une chambre de compression entre le conteneur extérieur et la poche intérieure, un orifice d'injection de gaz de vidage sous pression dans la chambre de compression étant pourvu sur ledit conteneur extérieur,
▪ le tube d'emplissage et le tube de vidage traversant le réceptacle extérieur par des liaisons étanches, fixes à demeure,
▪ les capacités de déformation respectivement de la poche intérieure et du conteneur extérieur étant choisies de sorte que lorsque l'on injecte le gaz de vidage sous pression dans la chambre de compression, la poche intérieure soit comprimé et que le fluide biopharmaceutique qui s'y trouve en soit vidé sous pression par la sortie de vidage,
▪ le réceptacle extérieur et la poche intérieure formant un tout cohérent à usage unique.

Le dispositif selon cette réalisation est tel que :
▪ la poche intérieure est apte et destinée à recevoir une quantité au moins égale à de l'ordre de 10 l de fluide biopharmaceutique, et le réceptacle extérieur déployé a une contenance d'au moins 40 litres,
▪ il comprend un tronçon de tube d'emplissage et un tronçon de tube de vidage situés entre le tronçon d'extrémité de bord de la poche intérieure dont ils sont adjacents et le tronçon d'extrémité de bord de la paroi du réceptacle extérieur qu'ils traversent par des liaisons étanches, fixes et à demeure, le tronçon d'extrémité de bord de la poche intérieure et le tronçon d'extrémité de bord de la paroi du réceptacle extérieur étant disposés en vis-à-vis et écartés l'un de l'autre, le bord de la poche intérieure étant écarté du bord du réceptacle extérieur
▪ il comprend également un moyen intégré de purge du gaz emplissant la ligne d'emplissage avant emplissage avec le fluide biopharmaceutique de sorte que ce gaz ne pénètre pas dans la poche intérieure, le dispositif étant spécialement adapté à la réception puis au vidage sous pression contrôlée par un laboratoire d'élaboration de produits pharmaceutiques d'un fluide biopharmaceutique en grande quantité, au moins égale à de l'ordre de 10 l, en vue d'un traitement ultérieur tel que filtration, formulation finale, remplissage de conteneurs de plus petite contenance.

Selon une réalisation, le conteneur extérieur comprend essentiellement, en particulier est constitué par, le réceptacle extérieur. En particulier, le réceptacle extérieur est une poche extérieure souple non extensible ou extensible avec une capacité d'expansion limitée, ou une coquille rigide ou semi-rigide.

Selon une réalisation, le conteneur extérieur comprend le réceptacle extérieur étant une poche extérieure souple, le cas échéant extensible, et un moyen de contention extérieur apte à recevoir la poche extérieure et ayant pour fonction de limiter la capacité d'expansion de la poche extérieure lorsque l'on injecte le gaz de vidage sous pression dans la chambre de compression. Par exemple, le moyen de contention comprend deux grandes parois rigides parallèles entre elles et espacées l'une de l'autre, en particulier d'espacement fixé, entre lesquelles vient se placer la poche extérieure comprenant deux grandes parois opposées et l'espace libre à la périphérie des deux grandes parois rigides fait fonction de passage pour la poche extérieure en vue de son placement ou en vue de son enlèvement entre les deux grandes parois. Le cas échéant, le moyen de contention comprend également une ou plusieurs parois de champ rigides, reliant rigidement les deux grandes parois.

Selon une réalisation, le dispositif, en outre, comporte ou est apte à être associé à, un moyen apte à ce que, au moins lors du vidage, l'orifice de vidage est situé vers la partie inférieure de la poche intérieure, en particulier la partie la plus inférieure de la poche intérieure qui, par exemple, est soit un moyen de suspension du dispositif vers l'opposé de l'orifice de vidage soit un moyen d'inclinaison du moyen de contention que comprend le conteneur extérieur.

Selon une réalisation, le moyen intégré de purge du gaz emplissant la ligne d'emplissage avant emplissage avec le fluide biopharmaceutique est une poche de vidage initial du gaz emplissant la ligne d'emplissage, associée par une connexion fluidique adventice au tube d'emplissage, à proximité de la poche intérieure, un dispositif d'ouverture/fermeture étant prévu sur la connexion fluidique adventice et un dispositif d'ouverture/fermeture étant prévu sur le tube d'emplissage au voisinage de la liaison avec la connexion fluidique adventice et entre celle-ci et la poche intérieure.

Selon une réalisation, le tube d'emplissage et le tube de vidage traversent en s'étendant de part et d'autre la paroi du réceptacle extérieur par des liaisons étanches et fixes à demeure par soudage ou analogue formées sur un tronçon d'extrémité de bord de cette paroi par deux zones en vis-à-vis de cette paroi, d'une part à plat l'une contre l'autre, d'autre part emprisonnant entre elles en épousant le tube d'emplissage et le tube de vidage.

Selon une réalisation, la paroi de la poche intérieure comporte un tronçon d'extrémité de bord où sont situés au voisinage l'un de l'autre l'orifice d'emplissage et l'orifice de vidage, alors que le tube d'emplissage et le tube de vidage sont situés au voisinage l'un de l'autre.

Selon une réalisation, le tube d'emplissage et le tube de vidage traversent en s'étendant du côté vers l'extérieur la paroi de la poche intérieure par des liaisons étanches et fixes à demeure par soudage ou analogue formées sur un tronçon d'extrémité de bord de cette paroi par deux zones en vis-à-vis de cette paroi, d'une part à plat l'une contre l'autre, d'autre part emprisonnant entre elles en épousant le tube d'emplissage et le tube de vidage.

Selon une réalisation, le tube d'emplissage et le tube de vidage traversent en s'étendant de part et d'autre la paroi du réceptacle extérieur sur un tronçon d'extrémité de bord de cette paroi et traversent en s'étendant du côté vers l'extérieur la paroi de la poche intérieure sur un tronçon d'extrémité de bord de cette paroi, le tronçon d'extrémité de bord de la paroi du réceptacle extérieur et le tronçon d'extrémité de bord de la poche intérieure s'étendant globalement parallèlement l'un à l'autre et étant situés au voisinage l'un de l'autre.

Selon une réalisation, le tube d'emplissage et le tube de vidage traversent en s'étendant de part et d'autre la paroi du réceptacle extérieur sur un tronçon d'extrémité de bord de cette paroi et traversent en s'étendant du côté vers l'extérieur la paroi de la poche intérieure sur un tronçon d'extrémité de bord de cette paroi, le tronçon d'extrémité de bord de la paroi du réceptacle extérieur et le tronçon d'extrémité de bord de la poche intérieure étant disposés en vis-à-vis, le tronçon de tube d'emplissage et le tronçon de tube de vidage situés entre le tronçon d'extrémité de bord de la paroi du réceptacle extérieur et le tronçon d'extrémité de bord de la poche intérieure étant autoportants et portant la poche intérieure.

Selon une réalisation, l'orifice d'injection de gaz de vidage sous pression, en particulier auquel est associé un tube d'injection ayant une entrée d'injection, est ménagé dans la paroi du réceptacle extérieur avec une liaison étanche et fixe à demeure par soudage ou analogue formée sur un tronçon d'extrémité de bord de cette paroi par deux zones en vis-à-vis de cette paroi, d'une part à plat l'une contre l'autre, d'autre part ménageant entre elles l'orifice d'injection, en particulier emprisonnant entre elles en épousant le tube d'injection.

Selon des réalisations possibles, la paroi du réceptacle extérieur comporte un tronçon d'extrémité de bord où sont situés au voisinage l'un de l'autre, ou les uns des autres, le tube d'emplissage, le tube de vidage, l'orifice d'injection, en particulier le tube d'injection. Et, à l'entrée d'emplissage et/ou à la sortie de vidage et/ou à l'orifice ou l'entrée d'injection est/sont associé/s un/des dispositif/s d'ouverture/fermeture et/ou un/des dispositif/s de raccordement fluidique.

Selon une réalisation, hormis l'éventuel fluide biopharmaceutique, l'enceinte intérieure de la poche intérieure est vide, en particulier de tube.

Selon une réalisation, hormis le tronçon de tube d'emplissage et le tronçon de tube de vidage situés entre le tronçon d'extrémité de bord de la paroi du réceptacle extérieur et le tronçon d'extrémité de bord de la poche intérieure disposés en vis-à-vis, la poche intérieure est montée libre dans le réceptacle extérieur.

Selon une réalisation, lorsque du gaz de vidage est injecté dans la chambre de compression, la paroi de la poche intérieure et la paroi du réceptacle extérieur la chambre de compression sont écartée l'une de l'autre sur tout ou substantiellement toute leur périphérie latérale.

Selon une réalisation, la poche intérieure a une paroi comprenant deux grandes portions de paroi opposées l'une à l'autre et le réceptacle extérieur est une poche dont la paroi comprend deux grandes portions de paroi opposées l'une à l'autre, de sorte que lorsque la poche intérieure et la poche du réceptacle extérieur sont vides, les parois respectives, ainsi que les poches elles-mêmes peuvent être pliées à plat et conformées en nappe.

Selon une réalisation, le réceptacle extérieur est au moins pour partie transparent de sorte à permettre de visualiser à travers sa paroi la poche intérieure.

Selon une réalisation, la poche intérieure déployée a une contenance comprise entre 8 litres et 60 litres, en particulier de l'ordre de 10 à 50 litres, alors que le réceptacle extérieur déployé a une contenance au moins égale à celle la poche intérieure déployée, notamment au moins égale à de l'ordre de 50 litres pour une poche intérieure déployée de contenance de l'ordre de 10 litres.

Selon une réalisation, le dispositif de réception puis de vidage sous pression contrôlée d'un fluide biopharmaceutique est avec un moyen avec perte de charge tel qu'un filtre associé en communication fluidique avec le tube de vidage ou la sortie de vidage de l'enceinte du fluide biopharmaceutique.

Selon une réalisation, le tube de vidage ou la sortie de vidage de l'enceinte du fluide biopharmaceutique est exempt d'une pompe, telle qu'une pompe péristaltique.

Selon un deuxième aspect, l'invention a pour objet un système pour la réception et le transfert sous pression contrôlée d'un fluide biopharmaceutique, comprenant :
▪ un dispositif de réception puis de vidage sous pression contrôlée d'un fluide biopharmaceutique tel qu'il vient d'être décrit, en particulier avec un moyen avec perte de charge tel qu'un filtre associé en communication fluidique avec le tube de vidage ou la sortie de vidage de l'enceinte du fluide biopharmaceutique,
▪ un moyen apte et destiné à délivrer un gaz de vidage sous pression ayant une ligne d'injection de gaz de vidage sous pression, apte à être associé en communication fluidique ou associé en communication fluidique avec l'orifice ou l'entrée d'injection de gaz de vidage sous pression dudit dispositif,
▪ et un moyen de contrôle et de commande de la pression du gaz de vidage sous pression dans la ligne d'injection de gaz de vidage sous pression.

Selon les réalisations, le moyen apte et destiné à délivrer un gaz de vidage sous pression délivre le gaz de vidage sous une pression au moins égale à 70 mbar, plus particulièrement au moins égale à 80 mbar, plus particulièrement au moins égale à 100 mbar, plus particulièrement au moins égale à 200 mbar, plus particulièrement au moins égale à 300 mbar et/ou sous une pression au plus égale à 600 mbar, plus particulièrement au plus égale à 500 mbar.

Selon une réalisation, le système pour la réception et le transfert sous pression contrôlée d'un fluide biopharmaceutique est exempt d'une pompe, telle qu'une pompe péristaltique, associée au tube de vidage ou la sortie de vidage de l'enceinte du fluide biopharmaceutique.

Selon un troisième aspect, l'invention a pour objet un procédé pour la réception et le transfert sous pression contrôlée d'un fluide biopharmaceutique, dans lequel :
▪ on dispose d'un système pour la réception et le transfert sous pression contrôlée d'un fluide biopharmaceutique tel qu'il vient d'être décrit alors à l'état vide de fluide biopharmaceutique et de gaz de vidage sous pression, et de fluide biopharmaceutique à réceptionner et à transférer sous pression contrôlée,
▪ lorsque l'on souhaite réceptionner du fluide biopharmaceutique dans le dispositif, d'abord on met en oeuvre le moyen intégré de purge du gaz emplissant la ligne d'emplissage et ainsi on purge le gaz emplissant la ligne d'emplissage, ensuite on emplit l'enceinte intérieure de la poche intérieure de fluide biopharmaceutique, via l'entrée d'emplissage puis on amène l'entrée d'emplissage à l'état fermé, la sortie de vidage étant à l'état fermé,
et on laisse le fluide biopharmaceutique dans l'enceinte intérieure de la poche intérieure autant que souhaité,
▪ et, lorsque l'on souhaite transférer sous pression contrôlée le fluide biopharmaceutique depuis l'enceinte intérieure :
   ∘ on associe en communication fluidique la ligne d'injection de gaz de vidage sous pression et l'entrée d'injection de gaz de vidage sous pression du réceptacle extérieur et on amène la sortie de vidage à l'état ouvert,
   ∘ puis on injecte le gaz de vidage sous pression dans la chambre de compression entre le réceptacle extérieur et la poche intérieure, de sorte à comprimer la poche intérieure et à vider de celle-ci sous pression, le fluide biopharmaceutique qui s'y trouve.

Selon une réalisation, on associe en communication fluidique un moyen avec perte de charge tel qu'un filtre avec le tube de vidage ou la sortie de vidage de l'enceinte du fluide biopharmaceutique.

Selon une réalisation, lors du vidage, on place l'orifice de vidage vers la partie inférieure de la poche intérieure, en particulier la partie la plus inférieure de la poche intérieure.

Selon une réalisation, on injecte le gaz de vidage de sorte que la pression du fluide biopharmaceutique dans la sortie de vidage soit sensiblement constante durant tout le vidage.

Selon les réalisations, on délivre le gaz de vidage sous une pression au moins égale à 70 mbar, plus particulièrement au moins égale à 80 mbar, plus particulièrement au moins égale à 100 mbar, plus particulièrement au moins égale à 200 mbar, plus particulièrement au moins égale à 300 mbar et/ou sous une pression au plus égale à 600 mbar, plus particulièrement au plus égale à 500 mbar.

Selon une réalisation, dans laquelle on met en œuvre un moyen intégré de purge du gaz emplissant la ligne d'emplissage comprenant une poche de vidage initial, une connexion fluidique adventice au tube d'emplissage, un dispositif d'ouverture/fermeture sur la connexion fluidique adventice et un dispositif d'ouverture/fermeture sur le tube d'emplissage, pour purger le gaz emplissant la ligne d'emplissage, avant emplissage avec le fluide biopharmaceutique, le dispositif d'ouverture/fermeture sur le tube d'emplissage est fermé, puis alors que le dispositif d'ouverture/fermeture sur la connexion fluidique adventice est ouvert, on commence l'emplissage avec le fluide biopharmaceutique, et lorsque le fluide biopharmaceutique atteint la connexion fluidique adventice, on ferme le dispositif d'ouverture/fermeture sur la connexion fluidique adventice et on ouvre dispositif d'ouverture/fermeture sur le tube d'emplissage.

Selon des possibilités, on délivre le gaz de vidage et on vide la poche intérieure du fluide biopharmaceutique pendant une durée comprise entre 2 minutes et 10 minutes.

Selon une caractéristique, on vide la poche intérieure de la totalité du fluide biopharmaceutique.

Selon une caractéristique, une fois le transfert sous pression contrôlée du fluide biopharmaceutique effectué, on jette le dispositif utilisé, celui-ci étant à usage unique.

Selon une réalisation où l'on met en oeuvre un dispositif de réception puis de vidage sous pression contrôlée d'un fluide biopharmaceutique dans lequel le conteneur extérieur comprend une poche extérieure souple et un moyen de contention extérieur, dans lequel on place la poche extérieure dans le moyen de contention extérieur pour limiter la capacité d'expansion de la poche extérieure lorsque l'on injecte le gaz de vidage sous pression dans la chambre de compression.

On décrit maintenant brièvement les figures des dessins.

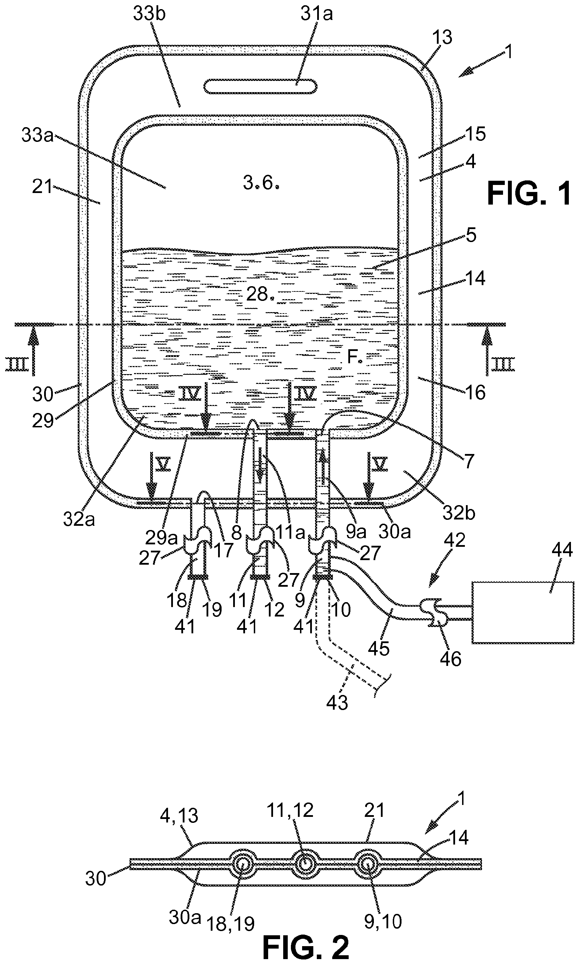

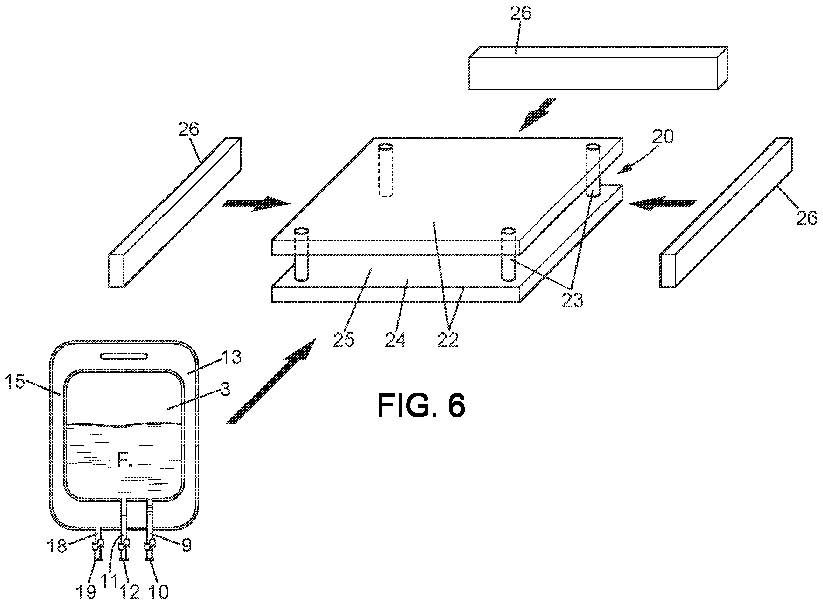

La figure 1 est une vue en élévation d'un dispositif de réception puis de vidage sous pression contrôlée d'un fluide biopharmaceutique selon l'invention, à plat, vide de fluide biopharmaceutique et de gaz de vidage, montrant sa poche intérieure, son conteneur extérieur comprenant un réceptacle extérieur étant une poche extérieure souple formant à elle seule le conteneur ou destinée à être associée à un conteneur extérieur, comme dans la figure 6, sa chambre de compression, le tube d'emplissage, le tube de vidage, l'orifice d'injection de gaz de vidage sous pression auquel est associé un tube d'injection, avec des dispositifs d'ouverture/fermeture, et un moyen intégré de purge, le réceptacle extérieur et la poche intérieure formant un tout cohérent à usage unique.
La figure 2 est une vue d'extrémité du dispositif représenté sur la figure 1.
La figure 3A est une vue en coupe à plus grande échelle du dispositif de la figure 1, à plat, vide de fluide biopharmaceutique et de gaz de vidage.
La figure 3B est une vue en coupe analogue à celle de la figure 3A, le dispositif étant en volume, dans la mesure où la poche intérieure est emplie de fluide biopharmaceutique, la chambre de compression étant vide de gaz de vidage.
La figure 3C est une vue en coupe analogue à celle des figures 3A et 3B, le dispositif étant en volume, dans la mesure où la poche intérieure est emplie de fluide biopharmaceutique et que la chambre de compression est emplie de gaz de vidage.
Les figures 4 et 5 sont deux vues en coupe, respectivement, selon les lignes IV-IV et V-V de la figure 1, illustrant la traversée de la poche intérieure par une liaison étanche et fixe à demeure du tube ici d'emplissage et la traversée du réceptacle extérieur par des liaisons étanches et fixes à demeure du tube d'emplissage, du tube de vidage et du tube d'injection.
La figure 6 est un schéma en éclaté du dispositif dans la réalisation où le conteneur extérieur comprend le réceptacle extérieur étant une poche extérieure et un moyen de contention extérieur apte à recevoir la poche extérieure et ayant pour fonction de limiter la capacité d'expansion de la poche extérieure lorsque l'on injecte le gaz de vidage sous pression dans la chambre de compression, le moyen intégré de purge ayant été omis de la figure.
La figure 7 est un schéma selon une coupe verticale illustrant le dispositif de la figure 6 monté avec la poche intérieure dans le moyen de contention, avec un moyen apte à ce que l'orifice de vidage est situé vers la partie inférieure de la poche intérieure étant un moyen d'inclinaison du moyen de contention que comprend le conteneur extérieur.
La figure 8 est un schéma illustrant un système pour la réception et le transfert sous pression contrôlée d'un fluide biopharmaceutique comprenant un dispositif tel que précédemment représenté et le procédé de mise en oeuvre le moyen intégré de purge étant représenté schématiquement sur la figure.

Ci-après un exposé détaillé de plusieurs modes de réalisation de l'invention assorti d'exemples et de référence aux dessins.

L'invention a pour objet un dispositif 1 de réception puis de vidage sous pression contrôlée d'un fluide biopharmaceutique (ledit dispositif 1 étant dénommé par la suite « dispositif »), un système 2 pour la réception et le transfert sous pression contrôlée d'un fluide biopharmaceutique comprenant le dispositif 1 (ledit système 2 étant dénommé par la suite « système ») et un procédé pour la réception et le transfert sous pression contrôlée d'un fluide biopharmaceutique, dans lequel on dispose et on met en œuvre le système 2.

Le dispositif 1 comprend une poche intérieure 3 et un conteneur extérieur 4.

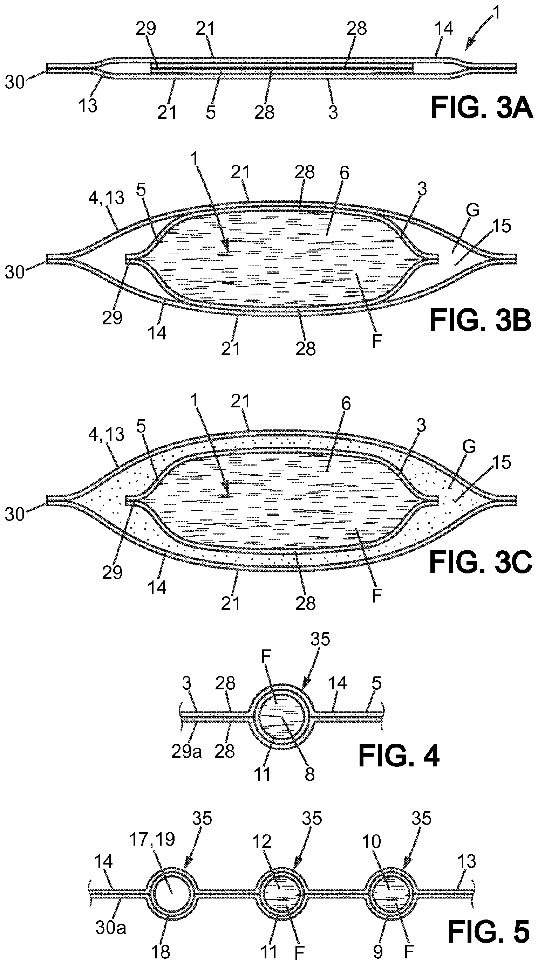

La poche intérieure 3 est formée à partir d'une paroi 5 en matière plastique. Comme sa paroi 5, la poche intérieure 3 est souple et étanche. La poche intérieure 3 et la paroi 5 forment et délimitent une enceinte intérieure 6, pouvant être à plat (figure 3A) ou déployée en volume (figures 3B et 3C) et qui est apte et destinée à recevoir du fluide biopharmaceutique F.

La poche intérieure 3 et la paroi 5 sont pourvues d'un orifice, c'est-à-dire d'un passage, d'emplissage 7 avec le fluide biopharmaceutique F et d'un orifice, c'est-à-dire d'un passage, de vidage 8 du fluide biopharmaceutique F. A l'orifice d'emplissage 7 et à l'orifice de vidage 8 de la poche intérieure 3 et de la paroi 5, sont associés par des liaisons étanches et fixes à demeure, respectivement un tube d'emplissage 9 ayant à l'opposé une entrée d'emplissage 10 de l'enceinte 6 avec le fluide biopharmaceutique F et un tube de vidage 11 ayant à l'opposé une sortie de vidage 12 de l'enceinte 6 du fluide biopharmaceutique F.

Par « liaison étanche et fixe à demeure », on entend une structure telle que la paroi 5 de la poche intérieure 3 et le tube 9, 11, en communication fluidique avec l'orifice 7, 8, sont associés entre eux de sorte, à la fois, à ne pas permettre un passage entre eux, notamment pour le fluide biopharmaceutique F ou un gaz ou de possibles contaminants et à former un tout solidaire indissociable.

Par « tube », on entend une structure creuse de longueur plus ou moins longue ou courte, le terme incluant également un simple port.

Le conteneur extérieur 4 comprend à tout le moins un réceptacle extérieur 13 formé à partir d'une paroi 14 en matière plastique. Comme sa paroi 14, le réceptacle extérieur 13 est souple et étanche. Le réceptacle 13 et la paroi 14 forment et délimitent une enceinte extérieure 15, pouvant également être à plat (figure 3A) ou déployée en volume (figures 3B et 3C).

Le réceptacle extérieur 13 du conteneur extérieur 4 est apte et destiné à recevoir la poche intérieure 3 (et donc l'enceinte intérieure 6) dans son entièreté. Ainsi, de fait, la poche intérieure 3 (et donc l'enceinte intérieure 6) est placée en totalité dans le, c'est-à-dire à l'intérieur du, réceptacle extérieur 13 et l'enceinte extérieure 15, ou symétriquement, le réceptacle 13 est placé de sorte à entourer à l'extérieur la poche intérieure 3 (et donc l'enceinte intérieure 6).

Une chambre, dite chambre de compression 16, est ménagée dans l'espace situé entre le réceptacle extérieur 13 du conteneur extérieur 4 et la poche intérieure 3.

Par suite, le réceptacle extérieur 13 est plus grand que la poche intérieure 3 ou symétriquement la poche intérieure 3 est plus petite que le réceptacle 13. Cela est vrai quand la poche intérieure 3 est vide de fluide biopharmaceutique F et la chambre de compression 16 du réceptacle extérieur 13 vide de gaz de vidage. Cela est vrai également quand la poche intérieure 3 est emplie de fluide biopharmaceutique F et la chambre de compression 16 du réceptacle extérieur 13 vide ou bien emplie de gaz de vidage.

L'entrée d'emplissage 10 et la sortie de vidage 12 associées à la poche intérieure 3 sont situées à l'extérieur du réceptacle extérieur 13, de sorte à être accessibles.

Le réceptacle extérieur 13 du conteneur extérieur 4 est pourvu d'un orifice, c'est-à-dire d'un passage, d'injection 17 de gaz de vidage sous pression G dans la chambre de compression 16, en communication fluidique avec la chambre de compression 16.

A l'orifice d'injection 17 du réceptacle extérieur 13 du conteneur extérieur 4 et de la paroi 14, est, dans la réalisation représentée, associés par une liaison étanche et fixe à demeure (cette expression devant être comprise comme exposé précédemment), un tube d'injection 18 (ce terme devant être compris comme exposé précédemment) ayant à l'opposé une entrée d'injection 19 du gaz de vidage G dans la chambre de compression 16.

Les qualificatifs « intérieur » et « extérieur », appliqué, respectivement à la poche 3 et à ses parties constitutives et au conteneur 4 et au réceptacle 13 reflètent le fait que le réceptacle 13 entoure à l'extérieur la poche 3 placée en totalité dans le, c'est-à-dire à l'intérieur du, réceptacle 13.

Les traversées du réceptacle extérieur 13 par le tube d'emplissage 9 et le tube de vidage 10 sont réalisées par des liaisons étanches et fixes à demeure (cette expression devant être comprise comme exposé précédemment).

Les capacités de déformation respectivement de la poche intérieure 3 et du réceptacle extérieur 13 sont choisies de sorte que lorsque l'on injecte le gaz de vidage sous pression G dans la chambre de compression 16, la poche intérieure 3 soit comprimé, de sorte que le fluide biopharmaceutique F qui s'y trouve en soit vidé sous pression par la sortie de vidage 12.

La poche intérieure 3 et le réceptacle extérieur 13 sont aptes à se trouver dans deux états extrêmes:
- un état (qualifié de vide) où la poche intérieure 3 et le réceptacle extérieur 13 présentent l'un et l'autre un volume intérieur faible, en particulier proche de zéro aux nécessités constructives près. Cet état est celui où la poche intérieure 3 et le réceptacle extérieur 13 sont vides de fluide biopharmaceutique F et de gaz de vidage G. Dans cet état, comme représenté sur les figures 1, 2 et 3A, la poche intérieure 3 et le réceptacle extérieur 13 peuvent être pliées à plat et conformés en nappe. Cet état est celui du dispositif 1 une fois réalisé et avant mise en oeuvre ou au début même de la mise en œuvre. Par exemple, c'est l'état du dispositif 1 stocké ou transporté.

- un état (qualifié d'empli) où la poche intérieure 3 et le réceptacle extérieur 13 sont mis en volume. Cet état est celui où la poche intérieure 3 est emplie de fluide biopharmaceutique F et le réceptacle extérieur 13 empli de gaz de vidage G, comme représenté sur la figure 3C. Cet état est celui du dispositif 1 lors de sa mise en œuvre. Dans cet état, la poche intérieure 3 et le réceptacle extérieur 13 sont configurés de sorte que la quantité souhaitée de fluide biopharmaceutique F emplit l'enceinte intérieure 6 et que la quantité appropriée de gaz de vidage G sous pression remplisse la chambre de compression 16.

Comme représenté sur la figure 3B, il est possible que le dispositif 1 se trouve également dans un état intermédiaire dans lequel la poche intérieure 3 est mise en volume, étant emplie de fluide biopharmaceutique F, alors que le réceptacle extérieur 13 n'est pas empli de gaz de vidage G, de sorte que bien que mis en volume, ce peut n'être que partiel et moindre que dans la situation représentée sur la figure 3C.

Par « capacité de déformation », on entend à la fois que la poche intérieure 3 et le réceptacle extérieur 13 sont aptes, chacun pour ce qui le concerne, à se trouver dans ces deux états extrêmes, et qu'ils sont aptes à passer de l'état vide à l'état empli lors de la mise en œuvre du dispositif 1.

Cette capacité de déformation relève en premier lieu de la conformation de la poche intérieure 3 et du réceptacle extérieur 13, typiquement de sorte à passer d'une conformation pliée à plat en nappe à une conformation en volume.

Cette capacité de déformation peut relever, en second lieu, d'une capacité de déformation intrinsèque des parois 5 et 14 de la poche intérieure 3 et du réceptacle extérieur 13, du fait qu'elles peuvent présenter une certaine capacité d'extension, notamment élastique. Mais, il doit être compris que le réceptacle extérieur 13 doit toujours remplir une fonction de contention extérieure de la poche intérieure 3, ce qui impose que la capacité d'expansion de la poche extérieure 13 lorsque l'on injecte le gaz de vidage sous pression G dans la chambre de compression 16 soit limitée, notamment très faible. Cela peut être obtenu par un choix adéquat du matériau ou du complexe formant la paroi 14 du réceptacle extérieur 13, ou par l'intégration à la paroi 14 du réceptacle extérieur 13 de moyens adapté, tels que rainures de renforcement.

Avec la structure qui vient d'être décrite, le réceptacle extérieur 13 et la poche intérieure 3 forment un tout cohérent à usage unique.

Dans une première réalisation qui peut être illustré par la figure 1, le conteneur extérieur 4 comprend essentiellement, et en particulier est constitué par, le réceptacle extérieur 13. Dans cette première réalisation, le réceptacle extérieur 13 peut, dans une première variante, être une poche extérieure 13 qui est souple, et soit est non extensible soit est extensible avec une capacité d'expansion limitée. Dans une seconde variante, le réceptacle extérieur 13 peut être une coquille rigide ou semi-rigide. Dans ces deux variantes, la fonction de contention extérieure de la poche intérieure 3 est assurée en totalité par le réceptacle extérieur 13.

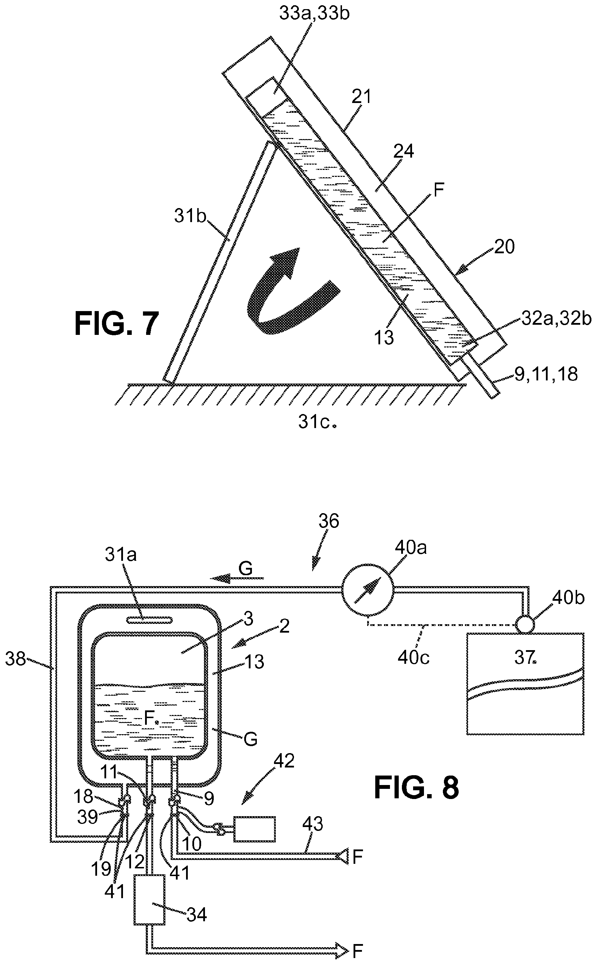

Dans une seconde réalisation, qui peut être illustré par la figure 1 en ce qui concerne le réceptacle extérieur 13, le conteneur extérieur 4 comprend non seulement le réceptacle extérieur 13 qui est une poche extérieure 13 souple, le cas échéant extensible, mais également un moyen de contention extérieur 20, rigide ou substantiellement rigide, celui-ci étant illustré par les figures 6 et 7. Dans ce cas, la fonction de contention extérieure de la poche intérieure 3 est assurée en tout ou partie par le moyen de contention extérieur 20.

Le réceptacle extérieur 13 et le moyen de contention extérieur 20 sont deux pièces structurellement distinctes, mais aptes à coopérer fonctionnellement. En effet, le moyen de contention extérieur 20 est destiné et apte à recevoir la poche extérieure 13. Il a pour fonction de limiter la capacité d'expansion de la poche extérieure 13 lorsque l'on injecte le gaz de vidage sous pression G dans la chambre de compression 16.

Avec la prévision d'un tel moyen de contention extérieur 20, l'on est sûr que lorsque l'on injecte le gaz de vidage sous pression G dans la chambre de compression 16, la poche intérieure 3 soit comprimé, de sorte que le fluide biopharmaceutique F qui s'y trouve en soit vidé sous pression par la sortie de vidage 12.

Dans une réalisation où la poche extérieure 13 comprend deux grandes portions de parois 21, opposées, le moyen de contention 20 peut comprendre deux grandes parois 22, rigides, parallèles entre elles, espacées l'une de l'autre (en particulier d'espacement fixé), par des entretoises périphériques 23, de sorte à ménager entre elles un espace 24. La poche extérieure 13 vient se loger dans cet espace 24, de sorte que ses deux grandes portions de parois 21 peuvent venir en appui contre les deux faces en vis-à-vis des parois 22.

L'espace libre 25 à la périphérie des deux parois 22 du moyen de contention 20 fait fonction de passage pour la poche extérieure 13 en vue de son placement ou en vue de son enlèvement entre les deux parois 22, c'est-à-dire de l'espace 24.

Le cas échéant, et comme représenté sur la figure 6 le moyen de contention 20 comprend également une ou plusieurs parois de chant rigides 26, apte à relier rigidement, de façon amovible, les deux grandes parois 22 en fermant totalement ou de façon substantielle l'espace libre 25, de sorte à empêcher la poche extérieure 13 de sortir ou saillir par l'espace libre 25 lorsque le gaz de vidage G est injecté dans la chambre de compression 16. Une telle paroi de chant 26 destinée à être placée du côté des tubes 9, 11 et 18 peut comprendre des trous ou échancrures adaptés au passage de ces tubes. Dans une variante, il n'est pas nécessaire de prévoir de telles parois de chant 26, la poche extérieure 13 étant conformée et constituée de sorte à ne pas sortir ou saillir de l'espace libre 25 lors de la mise en œuvre du dispositif 1.

La réalisation qui vient d'être décrite du moyen de contention 20 n'est aucunement exclusive d'autres. En particulier, le moyen de contention 20 peut être intégré de façon plus ou moins importante à la poche extérieure 13, ou avoir une structure autre que celle à parois 22, espace 24, espace 25, paroi 26 qui vient d'être décrite.

Dans tous les cas, le dispositif 1 est agencé de sorte que, lorsque l'on injecte le gaz de vidage sous pression G dans la chambre de compression 16, la poche intérieure 3 soit comprimée grâce au gaz de vidage sous pression G, de sorte que le fluide biopharmaceutique F qui s'y trouve en soit vidé sous pression par la sortie de vidage 12.

Selon une réalisation, à l'entrée d'emplissage 10, à la sortie de vidage 12, à l'entrée d'injection 19 est associé un dispositif d'ouverture/fermeture 27 (figure 1) et/ou un dispositif de raccordement fluidique 41, apte à être raccordé de façon fluidique, fixe et étanche avec, respectivement, une ligne d'emplissage 43, une ligne d'injection de gaz de vidage G sous pression 38 lorsque le dispositif 1 est incorporé au système 2 et mis en œuvre (voir figure 8). De tels dispositifs d'ouverture/fermeture et de raccordement fluidique 27, 41 sont connus de l'homme du métier des systèmes à poches et tubes pour l'industrie biopharmaceutique.

Dans la réalisation représentée, la poche intérieure 3 a une paroi 5 comprenant deux grandes portions de paroi 28, opposées l'une à l'autre, réunies de façon étanche et fixe à demeure, par soudage ou analogue, sur leur bord latéral périphérique commun qui est aussi le bord latéral périphérique 29 de la poche intérieure 3. De façon analogue, le réceptacle extérieur 13 est alors une poche extérieure 13 dont la paroi 14 comprend les deux grandes portions de paroi 21, comme il a été envisagé précédemment, opposées l'une à l'autre, réunies de façon étanche et fixe à demeure, par soudage ou analogue, sur leur bord latéral périphérique commun qui est aussi le bord latéral périphérique 30 du réceptacle ou poche extérieure 13. Dans ce cas, « réceptacle extérieur» et « poche extérieure » peuvent être considérés comme des expressions synonymes.

Avec une telle réalisation, lorsque la poche intérieure 3 et la poche extérieure 13 sont vides, les parois respectives 5, 14, ainsi que les poches 3, 13, elles-mêmes, peuvent être pliées à plat et conformées en nappe, comme représenté sur les figures 1, 2 et 3A (état vide), où les bords périphériques 29 et 30 ont des contours rectangulaires à coins arrondis (comme cela est connu de l'homme du métier pour des poches dans le domaine biopharmaceutique), le bord 29 de la poche intérieure 3 étant inclus dans le bord 30 de la poche extérieure 13 et les deux bords 29 et 30 disposés l'un par rapport à l'autre de façon écartée et au moins sensiblement parallèlement.

Lorsque la poche intérieure 3 est emplie de fluide biopharmaceutique F et la poche extérieure 13 emplie de gaz de vidage G qui y a été injecté (état empli), les deux poches 3, 13 sont en volume, les grandes portions 28 de la poche intérieure 3 peuvent ne pas être en contact avec les grandes portions 21 de la poche du réceptacle extérieur 13, mais par exemple seulement proches d'elles, le gaz de vidage G entourant alors totalement la poche intérieure 3 et les capacités de déformation de la poche intérieure 3 et du réceptacle extérieur 13 étant telles, relativement l'une par rapport à l'autre, que le gaz de vidage sous pression G comprime en tout état de cause de façon effective la poche intérieure 3, même si le réceptacle extérieur 13 est lui-même expansé. Cette réalisation est représentée en figure 3C.

Mais, il est également possible que les grandes portions 28 de la poche intérieure 3 viennent au moins en partie en appui (par leurs faces dirigées vers l'extérieur) contre les grandes portions 21 de la poche du réceptacle extérieur 13 (par leurs faces dirigées vers l'intérieur), la paroi 5 de la poche intérieure 3 et la paroi 14 de la poche extérieure 13 étant écartées l'une de l'autre sur tout ou substantiellement toute leur périphérie latérale (c'est-à-dire hors les grandes portions 28 et 21). Une telle configuration correspond à celle de la figure 3B, étant précisé qu'ici il y a du gaz de vidage G dans la chambre de compression, ce qui n'est pas le cas dans le contexte de la figure 3B.

Selon une réalisation, le réceptacle extérieur 13 (ou poche extérieure) est au moins pour partie transparent de sorte à permettre de visualiser à travers sa paroi 14 la poche intérieure 3. Le cas échéant, le moyen de contention extérieur 20 est également au moins pour partie transparent.

D'autre part, le dispositif 1 peut être pourvu d'un étiquetage pouvant être lu de l'extérieur du réceptacle extérieur 13.

Selon une réalisation, la poche intérieure 3 déployée a une contenance comprise entre 8 litres et 60 litres, en particulier de l'ordre de 10 à 50 litres, et ce en fonction des besoins et des applications.

Le réceptacle extérieur 13 déployé a une contenance au moins égale à celle la poche intérieure 3 déployée. Ainsi, et à titre illustratif, le réceptacle extérieur 13 déployé peut avoir une contenance au moins égale à de l'ordre de 50 litres pour une poche intérieure déployée de contenance de l'ordre de 10.

Selon une disposition constructive, illustrée par les figures 1 et 7, il est prévu que le dispositif 1 est tel qu'il comporte ou est apte à être associé à, un moyen 31a, 31b qui lui-même est apte à ce que, au moins lors du vidage du fluide biopharmaceutique F, l'orifice de vidage 8 est situé vers la partie inférieure 32a de la poche intérieure 3 et de l'enceinte intérieure 6 et, en particulier, vers la partie la plus inférieure. Cela vise à faire en sorte que, pour des raisons de sécurité, l'air qui se trouve dans la partie supérieure 33a de la poche intérieure 3 et de l'enceinte intérieure 6 ne puisse en sortir par l'orifice de vidage 8.

Selon une réalisation possible (figure 1), ce moyen 31a est un moyen de suspension 31a du dispositif 1 situé vers l'opposé de l'orifice de vidage 8, tel qu'un œillet de suspension prévu dans la partie supérieure 33b du réceptacle extérieur 13, ou éventuellement dans la partie supérieure 33a de la poche intérieure 3. Ainsi, le dispositif 1 peut être disposé verticalement avec l'orifice de vidage 8 vers le bas.

Selon une autre réalisation possible (figure 7), ce moyen 31b est un moyen d'inclinaison 31b du moyen de contention 20, le dispositif 1 en comprenant un. Ce moyen d'inclinaison 31b peut comporter une ou plusieurs jambes, par exemple articulées, associées à une ou aux deux parois 22 et reposant à l'opposé sur une surface d'appui horizontale 31c. Ainsi, le dispositif 1 peut être disposé incliné sur l'horizontale avec l'orifice de vidage 8 vers le bas.

Ces deux réalisations du moyen 31a, 31b, aptes à ce que, au moins lors du vidage du fluide biopharmaceutique F, l'orifice de vidage 8 est situé vers la partie inférieure , en particulier la plus inférieure, 32a de la poche intérieure 3, ne sont pas exclusives d'autres.

Il est entendu que les termes « inférieur », « supérieur », « bas » se comprennent en relation avec le dispositif 1 disposé pour pouvoir fonctionner.

Le dispositif 1 comprend également un moyen intégré 42 de purge du gaz emplissant la ligne d'emplissage 43 avant emplissage avec le fluide biopharmaceutique de sorte que ce gaz ne pénètre pas dans la poche intérieure,

Dans une réalisation possible illustrée par la figure 1, le moyen intégré de purge 42 comprend d'abord une poche 44 de vidage initial du gaz emplissant la ligne d'emplissage 43 qui est associée par une connexion fluidique adventice 45 au tube d'emplissage 9, à proximité de la poche intérieure 3, par exemple à proximité de l'entrée d'emplissage 10 et du dispositif d'ouverture/fermeture 27 prévu sur le tube d'emplissage 9 au voisinage de l'entrée d'emplissage 10.

La poche 44 de vidage initial est d'une contenance telle qu'elle peut recevoir la totalité du gaz emplissant la ligne d'emplissage. Une telle poche peut être flexible et en matière plastique, à l'instar de la poche intérieure 3. Le moyen intégré de purge 42 comprend ensuite un dispositif d'ouverture/fermeture 46 sur la connexion fluidique adventice 45 entre la poche 44 et le tube d'emplissage 9. Comme déjà indiqué, il est prévu également un dispositif d'ouverture/fermeture 27 sur le tube d'emplissage au voisinage de la liaison avec la connexion fluidique adventice 45 et entre celle-ci et la poche intérieure 3.

Dans la réalisation représentée, la poche 44 de vidage est située à l'extérieur du réceptacle 13.

Dans une application du dispositif 1 et du système 2 en vue d'une étape de filtration ou analogue, suivie d'une étape de formulation finale ou de remplissage de petits conteneurs, il est prévu un moyen avec perte de charge 34, tel qu'un filtre, associé en communication fluidique avec le tube de vidage 11 ou la sortie de vidage 12 de l'enceinte 6 du fluide biopharmaceutique F.

Avec les pressions du gaz de vidage G envisagées, il est possible de vider une poche intérieure 3 telle que celle décrite de la quantité de fluide biopharmaceutique F qui se trouvait dans son enceinte 6 et de lui faire traverser le filtre 34, en un temps assez court, et sans la nécessité de prévoir une pompe, telle qu'une pompe péristaltique, associée au tube de vidage 11 ou à la sortie de vidage 12. Ainsi, le dispositif 1 a aussi pour caractéristique que le tube de vidage 11 ou la sortie de vidage 12 peut être exempt d'une pompe, telle qu'une pompe péristaltique.

Dans la réalisation représentée, où les deux bords périphériques 29 et 30 ont des contours rectangulaires, ceux-ci peuvent comprendre deux tronçons d'extrémité 29a et 30a, situés respectivement vers les parties dites inférieures 32a et 32b de la poche intérieure 2 et du réceptacle extérieur 13. Ces deux tronçons d'extrémité de bord 29a, 30a s'étendent globalement parallèlement l'un à l'autre et étant situés au voisinage l'un de l'autre, disposés en vis-à-vis et écartés l'un de l'autre.

Le tube d'emplissage 9 et le tube de vidage 11 traversent tout d'abord la paroi 5 de la poche intérieure 3 en s'étendant du côté vers l'extérieur (c'est-à-dire vers le tronçon d'extrémité de bord 30a du réceptacle extérieur 13) par des liaisons étanches et fixes à demeure 35 formées sur le tronçon d'extrémité de bord 29a. En particulier, l'orifice d'emplissage 7 et l'orifice de vidage 8 sont situés au voisinage l'un de l'autre sur le tronçon d'extrémité de bord 29a, le tube d'emplissage 9 et le tube de vidage 11 étant situés au voisinage l'un de l'autre.

Le tube d'emplissage 9 et le tube de vidage 11 traversent ensuite la paroi 14 du réceptacle extérieur 13 en s'étendant de part et d'autre par des liaisons étanches et fixes à demeure 35 formées sur le tronçon d'extrémité de bord 30a.

Par ailleurs, l'orifice d'injection 17, avec le tube d'injection 18, est ménagé dans, respectivement traverse la paroi 14 du réceptacle extérieur 13 avec une liaison étanche et fixe à demeure 35 formée également sur le tronçon d'extrémité de bord 30a.

Sur le tronçon d'extrémité de bord 30a sont situés au voisinage l'un de l'autre, ou les uns des autres, le tube d'emplissage 9, le tube de vidage 11, l'orifice d'injection 17, avec le tube d'injection 18.

Avec la disposition décrite, le dispositif 1 comprend un tronçon 9a de tube d'emplissage 9 et un tronçon 11a de tube de vidage 11 situés entre le tronçon d'extrémité de bord 29a de la poche intérieure 3 dont ils sont adjacents et le tronçon d'extrémité de bord 30a de la paroi 14 du réceptacle extérieur 13 qu'ils traversent par des liaisons étanches, fixes et à demeure 35, Ces tronçons 9a et 11a de tube d'emplissage 9 et de tube de vidage 11 sont par exemple autoportants et portent la poche intérieure 3 dans le réceptacle extérieur 13, sans la nécessité d'associer directement la poche intérieure 3 et le réceptacle extérieur 13 par exemple à l'endroit d'une liaison de leurs grandes parois, avec les inconvénients inhérents (difficulté de réalisation, risque de fuite, surépaisseur). En outre, la poche intérieure 3 est écartée du réceptacle extérieur 13 vers les orifices 7 et 8 et 17, ce qui ne gêne pas le passage du fluide biopharmaceutique F et celui du gaz de vidage G. Egalement, le gaz de vidage G entoure la poche intérieure 3 vers les orifices 7 et 8 vers les orifices 7 et 8. Toutes ces dispositions constructives concourent à l'efficacité et à l'efficience de la mise en œuvre du dispositif 1 et du système 2.

Une liaison étanche et fixe à demeure 35 telle que celle précédemment mentionnée peut être réalisée par soudage ou analogue en étant formée sur le tronçon d'extrémité de bord 29a, 30a concerné, par deux zones en vis-à-vis de la paroi concernée 5, 14 qui, d'une part sont à plat l'une contre l'autre, d'autre part ménagent entre elles un orifice tel que 7, 8, 17 et/ou emprisonnent entre elles en épousant un tube tel que 9, 11, 18.

Dans la réalisation du dispositif 1 vide représentée, hormis l'éventuel fluide biopharmaceutique F, l'enceinte intérieure 6 de la poche intérieure 5 est vide, en particulier de tube. Et, de façon analogue, hormis le tronçon 9a de tube d'emplissage 9 et le tronçon 11a de tube de vidage 11, la chambre de compression 16 est vide, la poche intérieure 3 étant pour le reste montée libre dans le réceptacle extérieur 13.

Le système 2 comprend tout d'abord le dispositif de réception puis de vidage sous pression contrôlée d'un fluide biopharmaceutique F 1 tel qu'il vient d'être décrit. En particulier, le dispositif 1 est à moyen avec perte de charge tel que le filtre 34.

Le système 2 comprend ensuite un moyen 36 apte et destiné à délivrer le gaz de vidage sous pression G incluant une source 37 de gaz de vidage sous pression G et une ligne d'injection 38 de gaz de vidage sous pression G, apte à être associé en communication fluidique ou associé en communication fluidique en sortie 39 avec l'orifice 17 ou l'entrée 19 d'injection de gaz de vidage sous pression G du dispositif 1.

Le système 2 comprend également un moyen 40a, 40b, 40c de contrôle et de commande de la pression du gaz de vidage sous pression G dans la ligne d'injection 38, de sorte à commander l'injection lorsque cela est souhaité et de commander l'injection avec la pression souhaitée. Un tel moyen 40a, 40b, 40c peut comprendre par exemple un manomètre 40a, une vanne réglable 40b et une ligne de commande 40c entre eux.

Le gaz de vidage sous pression G est délivré sous une pression au moins égale à 70 mbar et au plus égale à 600 mbar.

Plus particulièrement et selon les besoins, cette pression est au moins égale à 80 mbar, plus particulièrement au moins égale à 100 mbar, plus particulièrement au moins égale à 200 mbar, plus particulièrement au moins égale à 300 mbar. Et elle est plus particulièrement au plus égale à 500 mbar.

Le système 2 comprend également ou bien il est associé au système 2 la ligne d'emplissage 43, déjà mentionnée.

Le procédé pour la réception et le transfert sous pression contrôlée d'un fluide biopharmaceutique F selon l'invention est tel que l'on dispose au départ d'un système 2 tel que décrit, alors à l'état vide de fluide biopharmaceutique F et de gaz de vidage sous pression G. On dispose également de fluide biopharmaceutique F à réceptionner et à transférer sous pression contrôlée. Et grâce au moyen 36, on dispose de gaz de vidage sous pression G.

Lorsque l'on souhaite réceptionner du fluide biopharmaceutique F dans le dispositif 1, on met d'abord en œuvre le moyen intégré 42 de purge du gaz emplissant la ligne d'emplissage 43 et ainsi on purge le gaz emplissant la ligne d'emplissage 43.

Ensuite on emplit l'enceinte intérieure 6 de la poche intérieure 3 de fluide biopharmaceutique F, via l'entrée d'emplissage 10, puis on amène l'entrée d'emplissage 10 à l'état fermé, la sortie de vidage 12 étant de son côté à l'état fermé.

On peut laisser le fluide biopharmaceutique F dans l'enceinte intérieure 6 de la poche intérieure 3 autant que souhaité, par exemple pour le stockage, le transport, la manipulation.

Lorsque l'on souhaite transférer sous pression contrôlée le fluide biopharmaceutique F depuis l'enceinte intérieure 6 de la poche intérieure 3, l'on associe en communication fluidique la ligne d'injection 38 (et notamment sa sortie 39) de gaz de vidage sous pression G et l'entrée d'injection 19 de gaz de vidage sous pression G du réceptacle extérieur 13 et on amène la sortie de vidage 12 à l'état ouvert. Puis l'on injecte le gaz de vidage sous pression G dans la chambre de compression 16 entre le réceptacle extérieur 13 et la poche intérieure 3, de sorte à comprimer la poche intérieure 3 et à vider de celle-ci sous pression, le fluide biopharmaceutique F qui s'y trouve.

Afin de mettre en œuvre le moyen intégré de purge 42 qui comprend la poche de vidage initial 44, la connexion fluidique adventice 45, le dispositif d'ouverture/fermeture 46 et le dispositif d'ouverture/fermeture 27 sur le tube d'emplissage 9, pour purger le gaz emplissant la ligne d'emplissage 38, on procède comme ceci : avant emplissage du dispositif 1 avec le fluide biopharmaceutique F, le dispositif d'ouverture/fermeture 27 sur le tube d'emplissage 9 est fermé ; puis alors que le dispositif d'ouverture/fermeture 46 est ouvert, on commence l'emplissage avec le fluide biopharmaceutique F .

Et, lorsque le fluide biopharmaceutique F atteint la connexion fluidique adventice 45, on ferme le dispositif d'ouverture/fermeture 46 et on ouvre dispositif d'ouverture/fermeture 27 sur le tube d'emplissage 9.

Comme indiqué, l'on peut associer en communication fluidique un moyen avec perte de charge tel qu'un filtre 14 avec le tube de vidage 11 ou la sortie de vidage 12.

Egalement, lors du vidage, l'on peut placer l'orifice de vidage 8 vers la partie inférieure 32a, en particulier la partie la plus inférieure, de la poche intérieure 3.

Selon une réalisation, l'on injecte le gaz de vidage G de sorte que la pression du fluide biopharmaceutique F dans la sortie de vidage 12 soit sensiblement constante durant tout le vidage.

Avec la pression de gaz de vidage sous une pression G précédemment indiquée, l'on peut vider une poche intérieure 3 telle que celle décrite, en une durée comprise entre de l'ordre de 2 minutes et 10 minutes. L'on peut vider la poche intérieure 3 de la totalité du fluide biopharmaceutique F. Une fois le transfert sous pression contrôlée du fluide biopharmaceutique F effectué, l'on peut jeter le dispositif 1 utilisé, celui-ci étant à usage unique.

Dans le cas où l'on met en œuvre un dispositif 1 dans lequel le conteneur extérieur 4 comprend une poche extérieure souple 13 et un moyen de contention extérieur 20, l'on place la poche extérieure 13 dans le moyen de contention 29 pour limiter la capacité d'expansion de la poche extérieure 13 lorsque l'on injecte le gaz de vidage sous pression G dans la chambre de compression 16.

## Revendications

1. Dispositif (1) de réception puis de vidage sous pression contrôlée par un laboratoire d'élaboration de produits pharmaceutiques d'un fluide biopharmaceutique en grande quantité, au moins égale à de l'ordre de 10 l, en vue d'un traitement ultérieur tel que filtration, formulation finale, remplissage de conteneurs de plus petite contenance, du type comprenant :
▪ une poche intérieure (3) en matière plastique, souple et étanche, ayant une enceinte intérieure (6) apte et destinée à recevoir une quantité au moins égale à de l'ordre de 10 l de fluide biopharmaceutique et pourvue d'un orifice d'emplissage (7) avec le fluide biopharmaceutique et d'un orifice de vidage (8) du fluide biopharmaceutique et, associés de façon étanche à l'orifice d'emplissage (7) et à l'orifice de vidage (8), un tube d'emplissage (9) ayant une entrée d'emplissage (10) de l'enceinte (6) avec le fluide biopharmaceutique, apte à être associé à une ligne d'emplissage (43) du fluide biopharmaceutique, et un tube de vidage (11) ayant une sortie de vidage (12) de l'enceinte (6) du fluide biopharmaceutique, apte à être associé à une ligne de vidage du fluide biopharmaceutique,
▪ un conteneur extérieur (4) dans lequel est placée la poche intérieure (3), une chambre de compression (16) étant ménagée entre le conteneur extérieur (4) et la poche intérieure (3) dont l'entrée d'emplissage (10) et la sortie de vidage (12) sont situées à l'extérieur du conteneur extérieur (4), un orifice d'injection (17) de gaz de vidage sous pression dans la chambre de compression (16) étant pourvu sur ledit conteneur extérieur (4),
▪ des traversées étanches du conteneur extérieur (4) par le tube d'emplissage (9) et le tube de vidage (11),
▪ les capacités de déformation respectivement de la poche intérieure (3) et du conteneur extérieur (4) étant choisies de sorte que lorsque l'on injecte le gaz de vidage sous pression dans la chambre de compression (16), la poche intérieure (3) soit comprimé et que le fluide biopharmaceutique qui s'y trouve en soit vidé sous pression par la sortie de vidage (12),
**caractérisé en ce que** :
▪ le conteneur extérieur (4) comprend un réceptacle extérieur (13) en matière plastique, étanche, formant une enceinte extérieure dans laquelle est placée la poche intérieure (3), ménageant la chambre de compression (16), et comportant l'orifice d'injection (17) de gaz de vidage sous pression,
▪ le tube d'emplissage (9) et le tube de vidage (11) traversent le réceptacle extérieur (13) par des liaisons fixes à demeure (35), l'entrée d'emplissage (10) et la sortie de vidage (12) étant situées à l'extérieur du réceptacle extérieur (13),
▪ il comprend également un moyen intégré de purge (42) du gaz emplissant la ligne d'emplissage (43) avant emplissage avec le fluide biopharmaceutique de sorte que ce gaz ne pénètre pas dans la poche intérieure (3),
▪ le réceptacle extérieur (13) et la poche intérieure (3) forment un tout cohérent à usage unique.

2. Dispositif (1) de réception puis de vidage sous pression contrôlée d'un fluide biopharmaceutique selon la revendication 1 **caractérisé en ce que** :
▪ la poche intérieure (3) est apte et destinée à recevoir une quantité au moins égale à de l'ordre de 10 l de fluide biopharmaceutique, et le réceptacle extérieur (13) déployé a une contenance d'au moins 40 litres,
▪ il comprend un tronçon (9a) de tube d'emplissage (9) et un tronçon (11a) de tube de vidage (11) situés entre le tronçon d'extrémité de bord (29a) de la poche intérieure (3) dont ils sont adjacents et le tronçon d'extrémité de bord (30a) de la paroi (14) du réceptacle extérieur (13) qu'ils traversent par des liaisons étanches, fixes et à demeure, le tronçon d'extrémité de bord (29a) de la poche intérieure (3) et le tronçon d'extrémité de bord (30a) de la paroi (14) du réceptacle extérieur (13) étant disposés en vis-à-vis et écartés l'un de l'autre,
▪ il comprend également un moyen intégré de purge (42) du gaz emplissant la ligne d'emplissage (43) avant emplissage avec le fluide biopharmaceutique de sorte que ce gaz ne pénètre pas dans la poche intérieure (3), le dispositif (1) étant spécialement adapté à la réception puis au vidage sous pression contrôlée par un laboratoire d'élaboration de produits pharmaceutiques d'un fluide biopharmaceutique en grande quantité, au moins égale à de l'ordre de 10 l, en vue d'un traitement ultérieur tel que filtration, formulation finale, remplissage de conteneurs de plus petite contenance.

3. Dispositif (1) de réception puis de vidage sous pression contrôlée d'un fluide biopharmaceutique selon l'une quelconque des revendications 1 et 2, dans lequel le conteneur extérieur (4) comprend essentiellement, en particulier est constitué par, le réceptacle extérieur (13).

4. Dispositif (1) de réception puis de vidage sous pression contrôlée d'un fluide biopharmaceutique selon la revendication 3, comprenant en outre l'une des caractéristiques suivantes :
- le réceptacle extérieur (13) est une poche extérieure (13) souple non extensible ou extensible avec une capacité d'expansion limitée ;
- le réceptacle extérieur (13) est une coquille rigide ou semi-rigide.

5. Dispositif (1) de réception puis de vidage sous pression contrôlée d'un fluide biopharmaceutique selon l'une quelconque des revendications 1 et 2, dans lequel le conteneur extérieur (4) comprend le réceptacle extérieur (13) étant une poche extérieure (13) souple, le cas échéant extensible, et un moyen de contention extérieur (20) apte à recevoir la poche extérieure (13) et ayant pour fonction de limiter la capacité d'expansion de la poche extérieure (13) lorsque l'on injecte le gaz de vidage sous pression dans la chambre de compression (16).

6. Dispositif (1) de réception puis de vidage sous pression contrôlée d'un fluide biopharmaceutique selon la revendication 5, dans lequel le moyen de contention (20) comprend deux grandes parois rigides (22) parallèles entre elles et espacées l'une de l'autre, en particulier d'espacement fixé, entre lesquelles vient se placer la poche extérieure (13) comprenant deux grandes parois (21) opposées.

7. Dispositif (1) de réception puis de vidage sous pression contrôlée d'un fluide biopharmaceutique selon la revendication 6, comprenant en outre l'une et/ou l'autre des caractéristiques suivantes :
- l'espace libre (25) à la périphérie des deux grandes parois rigides (22) fait fonction de passage pour la poche extérieure (13) en vue de son placement ou en vue de son enlèvement entre les deux grandes parois (22) ;
- le moyen de contention (20) comprend également une ou plusieurs parois de champ (26), rigides, reliant rigidement les deux grandes parois (22).

8. Dispositif (1) de réception puis de vidage sous pression contrôlée d'un fluide biopharmaceutique selon l'une quelconque des revendications 1 à 7, qui, en outre, comporte ou est apte à être associé à, un moyen (31a, 31b, 31c) apte à ce que, au moins lors du vidage, l'orifice de vidage (8) est situé vers la partie inférieure de la poche intérieure (3), en particulier la partie la plus inférieure de la poche intérieure (3) ;
, et optionnellement dans lequel le moyen apte à ce que l'orifice de vidage (8) est situé vers la partie inférieure de la poche intérieure (3) est soit un moyen (31a) de suspension du dispositif (1) vers l'opposé de l'orifice de vidage (8) soit un moyen (31b, 31c) d'inclinaison du moyen de contention (20) que comprend le conteneur extérieur (4).

9. Dispositif (1) de réception puis de vidage sous pression contrôlée d'un fluide biopharmaceutique selon l'une quelconque des revendications 1 à 8, comprenant en outre l'une et/ou l'autre des caractéristiques suivantes :
- le moyen intégré de purge (42) du gaz emplissant la ligne d'emplissage (43) avant emplissage avec le fluide biopharmaceutique est une poche de vidage initial (44) du gaz emplissant la ligne d'emplissage (43), associée par une connexion fluidique adventice (45) au tube d'emplissage (9), à proximité de la poche intérieure (3), un dispositif (46) d'ouverture/fermeture étant prévu sur la connexion fluidique adventice (45) et un dispositif (27) d'ouverture/fermeture étant prévu sur le tube d'emplissage (9) au voisinage de la liaison avec la connexion fluidique adventice (45) et entre celle-ci et la poche intérieure (3) ;
- le tube d'emplissage (9) et le tube de vidage (11) traversent en s'étendant de part et d'autre la paroi (14) du réceptacle extérieur (13) par des liaisons étanches et fixes à demeure par soudage ou analogue formées sur un tronçon d'extrémité de bord de cette paroi par deux zones en vis-à-vis de cette paroi, d'une part à plat l'une contre l'autre, d'autre part emprisonnant entre elles en épousant le tube d'emplissage (9) et le tube de vidage (11) ;
- la paroi (5) de la poche intérieure (3) comporte un tronçon d'extrémité de bord (29a) où sont situés au voisinage l'un de l'autre l'orifice d'emplissage (7) et l'orifice de vidage (8), alors que le tube d'emplissage (9) et le tube de vidage (11) sont situés au voisinage l'un de l'autre ;
- le tube d'emplissage (9) et le tube de vidage (11) traversent en s'étendant du côté vers l'extérieur la paroi (5) de la poche intérieure (3) par des liaisons étanches et fixes à demeure par soudage ou analogue formées sur un tronçon d'extrémité de bord (29a) de cette paroi (5) par deux zones en vis-à-vis de cette paroi, d'une part à plat l'une contre l'autre, d'autre part emprisonnant entre elles en épousant le tube d'emplissage (9) et le tube de vidage (11).

10. Dispositif (1) de réception puis de vidage sous pression contrôlée d'un fluide biopharmaceutique selon l'une quelconque des revendications 1 à 9, comprenant en outre l'une et/ou l'autre des caractéristiques suivantes :
- le tube d'emplissage (9) et le tube de vidage (11) traversent en s'étendant de part et d'autre la paroi (14) du réceptacle extérieur (13) sur un tronçon d'extrémité de bord (30a) de cette paroi et traversent en s'étendant du côté vers l'extérieur la paroi (5) de la poche intérieure (3) sur un tronçon d'extrémité de bord de cette paroi, le tronçon d'extrémité de bord (30a) de la paroi (14) du réceptacle extérieur (13) et le tronçon d'extrémité de bord (29a) de la poche intérieure (3) s'étendant globalement parallèlement l'un à l'autre et étant situés au voisinage l'un de l'autre ;
- le tube d'emplissage (9) et le tube de vidage (11) traversent en s'étendant de part et d'autre la paroi (14) du réceptacle extérieur (13) sur un tronçon d'extrémité de bord de cette paroi (14) et traversent en s'étendant du côté vers l'extérieur la paroi (5) de la poche intérieure (3) sur un tronçon d'extrémité de bord (29a) de cette paroi (5), le tronçon d'extrémité de bord (30a) de la paroi (14) du réceptacle extérieur (13) et le tronçon d'extrémité de bord (29a) de la poche intérieure (3) étant disposés en vis-à-vis, le tronçon (9a) de tube d'emplissage (9) et le tronçon (11a) de tube de vidage (11) situés entre le tronçon d'extrémité de bord (30a) de la paroi (14) du réceptacle extérieur (13) et le tronçon d'extrémité de bord (29a) de la poche intérieure (3) étant autoportants et portant la poche intérieure (3) ;
- l'orifice d'injection (17) de gaz de vidage sous pression, en particulier auquel est associé un tube d'injection ayant une entrée d'injection, est ménagé dans la paroi (14) du réceptacle extérieur (13) avec une liaison étanche et fixe à demeure par soudage ou analogue formée sur un tronçon d'extrémité de bord de cette paroi par deux zones en vis-à-vis de cette paroi, d'une part à plat l'une contre l'autre, d'autre part ménageant entre elles l'orifice d'injection (17), en particulier emprisonnant entre elles en épousant le tube d'injection ;
- la paroi (14) du réceptacle extérieur (13) comporte un tronçon d'extrémité de bord où sont situés au voisinage l'un de l'autre, ou les uns des autres, le tube d'emplissage (9), le tube de vidage (11), l'orifice d'injection (17), en particulier le tube d'injection ;
- hormis le tronçon (9a) de tube d'emplissage (9) et le tronçon (11a) de tube de vidage (11) situés entre le tronçon d'extrémité de bord (30a) de la paroi (14) du réceptacle extérieur (13) et le tronçon d'extrémité de bord (29a) de la poche intérieure (3) disposés en vis-à-vis, la poche intérieure (3) est montée libre dans le réceptacle extérieur (13).

11. Dispositif (1) de réception puis de vidage sous pression contrôlée d'un fluide biopharmaceutique selon l'une quelconque des revendications 1 à 10, comprenant en outre l'une et/ou l'autre des caractéristiques suivantes :
- lorsque du gaz de vidage est injecté dans la chambre de compression (16), la paroi (5) de la poche intérieure (3) et la paroi (14) du réceptacle extérieur (13) de la chambre de compression (16) sont écartées l'une de l'autre sur tout ou substantiellement toute leur périphérie latérale ;
- la poche intérieure (3) a une paroi comprenant deux grandes portions de paroi opposées l'une à l'autre et le réceptacle extérieur (13) est une poche dont la paroi comprend deux grandes portions de paroi opposées l'une à l'autre, de sorte que lorsque la poche intérieure (3) et la poche du réceptacle extérieur (13) sont vides, les parois respectives, ainsi que les poches elles-mêmes peuvent être pliées à plat et conformées en nappe ;
- le réceptacle extérieur (13) est au moins pour partie transparent de sorte à permettre de visualiser à travers sa paroi la poche intérieure (3) ;
- la poche intérieure (3) déployée a une contenance comprise entre 8 litres et 60 litres, en particulier de l'ordre de 10 à 50 litres, alors que le réceptacle extérieur (13) déployé a une contenance au moins égale à celle la poche intérieure (3) déployée, notamment au moins égale à de l'ordre de 50 litres pour une poche intérieure (3) déployée de contenance de l'ordre de 10 ;
- le dispositif comporte un moyen avec perte de charge (34) tel qu'un filtre associé en communication fluidique avec le tube de vidage (11) ou la sortie de vidage (12) de l'enceinte (6) du fluide biopharmaceutique ;
- le tube de vidage (11) ou la sortie de vidage (12) de l'enceinte (6) du fluide biopharmaceutique est exempt d'une pompe, telle qu'une pompe péristaltique.

12. Système pour la réception et le transfert sous pression contrôlée d'un fluide biopharmaceutique, comprenant :
▪ un dispositif (1) de réception puis de vidage sous pression contrôlée d'un fluide biopharmaceutique selon l'une quelconque des revendications 1 à 11, en particulier avec un moyen avec perte de charge (34) tel qu'un filtre associé en communication fluidique avec le tube de vidage (11) ou la sortie de vidage (12) de l'enceinte (6) du fluide biopharmaceutique,
▪ un moyen apte et destiné à délivrer un gaz de vidage sous pression ayant une ligne d'injection de gaz de vidage sous pression, apte à être associé en communication fluidique ou associé en communication fluidique avec l'orifice ou l'entrée d'injection de gaz de vidage sous pression dudit Dispositif (1),
▪ et un moyen de contrôle et de commande de la pression du gaz de vidage sous pression dans la ligne d'injection de gaz de vidage sous pression.

13. Système pour la réception et le transfert sous pression contrôlée d'un fluide biopharmaceutique selon la revendication 12, comprenant en outre l'une et/ou l'autre des caractéristiques suivantes :
- le moyen apte et destiné à délivrer un gaz de vidage sous pression délivre le gaz de vidage sous une pression au moins égale à 70 mbar, plus particulièrement au moins égale à 80 mbar, plus particulièrement au moins égale à 100 mbar, plus particulièrement au moins égale à 200 mbar, plus particulièrement au moins égale à 300 mbar et/ou sous une pression au plus égale à 600 mbar, plus particulièrement au plus égale à 500 mbar ;
- le système est exempt d'une pompe, telle qu'une pompe péristaltique, associée au tube de vidage (11) ou la sortie de vidage (12) de l'enceinte (6) du fluide biopharmaceutique.

14. Procédé pour la réception et le transfert sous pression contrôlée d'un fluide biopharmaceutique, dans lequel :
▪ on dispose d'un système pour la réception et le transfert sous pression contrôlée d'un fluide biopharmaceutique selon l'une quelconque des revendications 12 à 13 alors à l'état vide de fluide biopharmaceutique et de gaz de vidage sous pression, et de fluide biopharmaceutique à réceptionner et à transférer sous pression contrôlée,
▪ lorsque l'on souhaite réceptionner du fluide biopharmaceutique dans le Dispositif (1), d'abord on met en œuvre le moyen intégré de purge (42) du gaz emplissant la ligne d'emplissage (43) et ainsi on purge le gaz emplissant la ligne d'emplissage (43), ensuite on emplit l'enceinte intérieure (6) de la poche intérieure (3) de fluide biopharmaceutique, via l'entrée d'emplissage (10) puis on amène l'entrée d'emplissage (10) à l'état fermé, la sortie de vidage (12) étant à l'état fermé, et on laisse le fluide biopharmaceutique dans l'enceinte intérieure (6) de la poche intérieure (3) autant que souhaité,
▪ et, lorsque l'on souhaite transférer sous pression contrôlée le fluide biopharmaceutique depuis l'enceinte intérieure (6) :
∘ on associe en communication fluidique la ligne d'injection de gaz de vidage sous pression et l'entrée d'injection de gaz de vidage sous pression du réceptacle extérieur (13) et on amène la sortie de vidage (12) à l'état ouvert,
∘ puis on injecte le gaz de vidage sous pression dans la chambre de compression (16) entre le réceptacle extérieur (13) et la poche intérieure (3), de sorte à comprimer la poche intérieure (3) et à vider de celle-ci sous pression, le fluide biopharmaceutique qui s'y trouve.

15. Procédé pour la réception et le transfert sous pression contrôlée d'un fluide biopharmaceutique selon la revendication 14, comprenant en outre l'une et/ou l'autre des caractéristique suivantes :
- on associe en communication fluidique un moyen avec perte de charge (34) tel qu'un filtre avec le tube de vidage (11) ou la sortie de vidage (12) de l'enceinte (6) du fluide biopharmaceutique ;
- lors du vidage, on place l'orifice de vidage (8) vers la partie inférieure de la poche intérieure (3), en particulier la partie la plus inférieure de la poche intérieure (3) ;
- on injecte le gaz de vidage de sorte que la pression du fluide biopharmaceutique dans la sortie de vidage (12) soit sensiblement constante durant tout le vidage ;
- on délivre le gaz de vidage sous une pression au moins égale à 70 mbar, plus particulièrement au moins égale à 80 mbar, plus particulièrement au moins égale à 100 mbar, plus particulièrement au moins égale à 200 mbar, plus particulièrement au moins égale à 300 mbar et/ou sous une pression au plus égale à 600 mbar, plus particulièrement au plus égale à 500 mbar ;
- on met en œuvre un moyen intégré de purge (42) du gaz emplissant la ligne d'emplissage (43) comprenant une poche de vidage initial (44), une connexion fluidique adventice (45) au tube d'emplissage (9), un dispositif (46) d'ouverture/fermeture sur la connexion fluidique adventice (45) et un dispositif (46) d'ouverture/fermeture sur le tube d'emplissage (9), et dans lequel pour purger le gaz emplissant la ligne d'emplissage (43), avant emplissage avec le fluide biopharmaceutique, le dispositif (46) d'ouverture/fermeture sur le tube d'emplissage (9) est fermé, puis alors que le dispositif (46) d'ouverture/fermeture sur la connexion fluidique adventice (45) est ouvert, on commence l'emplissage avec le fluide biopharmaceutique, et lorsque le fluide biopharmaceutique atteint la connexion fluidique adventice (45), on ferme le dispositif (46) d'ouverture/fermeture sur la connexion fluidique adventice (45) et on ouvre dispositif (46) d'ouverture/fermeture sur le tube d'emplissage (9) ;
- on met en œuvre un dispositif (1) de réception puis de vidage sous pression contrôlée d'un fluide biopharmaceutique dans lequel le conteneur extérieur (4) comprend une poche extérieure (13) souple et un moyen de contention extérieur (20), dans lequel on place la poche extérieure (13) dans le moyen de contention extérieur (20) pour limiter la capacité d'expansion de la poche extérieure (13) lorsque l'on injecte le gaz de vidage sous pression dans la chambre de compression (16).

## Patentansprüche

1. Vorrichtung (1) zur Aufnahme und anschließenden kontrollierten Entleerung unter Druck einer biopharmazeutischen Flüssigkeit in einer großen Menge in der Größenordnung von mindestens gleich 10 1 durch ein Labor zur Verarbeitung von biopharmazeutischen Produkten zur Weiterbehandlung, wie beispielsweise Filtern, Endformulierung, Befüllung von Behältern mit geringerem Inhalt, vom Typ, der umfasst:
▪ einen Innenbeutel (3) aus Kunststoff, der biegsam und dicht ist, eine Innenkammer (6), die dazu geeignet und bestimmt ist, eine Menge an biopharmazeutischer Flüssigkeit in der Größenordnung von mindestens gleich 10 1 aufzunehmen, und mit einer Öffnung (7) zum Füllen mit der biopharmazeutischen Flüssigkeit und mit einer Öffnung (8) zur Entleerung der biopharmazeutischen Flüssigkeit versehen ist, die auf dichte Weise mit der Füllöffnung (7) und mit der Entleerungsöffnung (8) verbunden sind, ein Füllrohr (9), das einen Eingang (10) zum Füllen der Kammer (6) mit der biopharmazeutischen Flüssigkeit aufweist, der geeignet ist, mit einer Leitung (43) zum Füllen mit der biopharmazeutischen Flüssigkeit verbunden zu werden, und ein Entleerungsrohr (11) aufweist, das einen Ausgang (12) zur Entleerung der biopharmazeutischen Flüssigkeit aus der Kammer (6) aufweist, der dazu geeignet ist, mit einer Leitung zur Entleerung der biopharmazeutischen Flüssigkeit verbunden zu sein,
▪ einen Außenbehälter (4), in dem der Innenbeutel (3) platziert ist, wobei eine Druckkammer (16) zwischen dem Außenbehälter (4) und dem Innenbeutel (3) aufgenommen ist, dessen Fülleingang (10) und Entleerungsausgang (12) sich auf der Außenseite des Außenbehälters (4) befinden, wobei der Außenbehälter (4) darauf mit einer Öffnung (17) zum Einpressen von Entleerungsgas unter Druck in die Druckkammer (16) versehen ist,
▪ dichte Durchführungen durch den Außenbehälter (4) über das Füllrohr (9) und das Entleerungsrohr (11),
▪ wobei die Verformungsfähigkeiten des Innenbeutels (3) beziehungsweise des Außenbehälters (4) derart gewählt sind, dass, wenn das Entleerungsgas unter Druck in die Druckkammer (16) eingepresst wird, der Innenbeutel (3) zusammengedrückt wird und die biopharmazeutische Flüssigkeit, die sich darin befindet, unter Druck über den Entleerungsausgang (12) daraus entleert wird,
**dadurch gekennzeichnet, dass**:
▪ der Außenbehälter (4) eine Außenaufnahme (13) aus Kunststoff umfasst, die dicht ist, eine Außenkammer bildet, in der der Innenbeutel (3) platziert ist, die Druckkammer (16) aufnimmt und die Öffnung (17) zum Einpressen von Entleerungsgas unter Druck umfasst,
▪ das Füllrohr (9) und das Entleerungsrohr (11) die Außenaufnahme (13) durch fest angebrachte Verbindungen (35) durchqueren, wobei der Fülleingang (10) und der Entleerungsausgang (12) sich auf der Außenseite der Außenaufnahme (13) befinden,
▪ sie auch ein integriertes Mittel (42) zum Ablassen des Gases, das die Füllleitung (43) füllt, vor dem Füllen mit der biopharmazeutischen Flüssigkeit umfasst, derart dass dieses Gas nicht in den Innenbeutel (3) eindringt,
▪ die Außenaufnahme (13) und der Innenbeutel (3) ein zusammenhängendes Ganzes zur einmaligen Verwendung bilden.

2. Vorrichtung (1) zur Aufnahme und anschließenden kontrollierten Entleerung unter Druck einer biopharmazeutischen Flüssigkeit nach Anspruch 1, **dadurch gekennzeichnet, dass**:
▪ der Innenbeutel (3) dazu geeignet und bestimmt ist, eine Menge an biopharmazeutischer Flüssigkeit in der Größenordnung von mindestens gleich 10 l aufzunehmen und die Außenaufnahme (13) im entfalteten Zustand einen Inhalt von mindestens 40 Litern aufweist,
▪ sie einen Abschnitt (9a) des Füllrohrs (9) und einen Abschnitt (11a) des Entleerungsohrs (11) umfasst, die sich zwischen dem Randendabschnitt (29a) des Innenbeutels (3), dem sie benachbart sind, und dem Randendabschnitt (30a) der Wand (14) der Außenaufnahme (13) befinden, die sie durch dichte, fest angebrachte Verbindungen durchqueren, wobei der Randendabschnitt (29a) des Innenbeutels (3) und der Randendabschnitt (30a) der Wand (14) des Außenbehälters (13) gegenüberliegend und voneinander beabstandet angeordnet sind,
▪ sie auch ein integriertes Mittel (42) zum Ablassen des Gases, das die Füllleitung (43) vor dem Füllen mit der biopharmazeutischen Flüssigkeit füllt, umfasst, derart dass dieses Gas nicht in den Innenbeutel (3) eindringt,
▪ wobei die Vorrichtung (1) eigens an die Aufnahme und anschließende kontrollierte Entleerung einer biopharmazeutischen Flüssigkeit in einer großen Menge, die mindestens gleich der Größenordnung von 10 l ist, durch ein Labor zur Verarbeitung biopharmazeutischer Produkte zur Weiterbehandlung, wie beispielsweise Filtern, Endformulierung, Befüllung von Behältern mit geringerem Inhalt, angepasst ist.

3. Vorrichtung (1) zur Aufnahme und anschließenden kontrollierten Entleerung unter Druck einer biopharmazeutischen Flüssigkeit nach einem der Ansprüche 1 und 2, wobei der Außenbehälter (4) im Wesentlichen die Außenaufnahme (13) umfasst und insbesondere aus ihr besteht.

4. Vorrichtung (1) zur Aufnahme und anschließenden kontrollierten Entleerung unter Druck einer biopharmazeutischen Flüssigkeit nach Anspruch 3, die ferner eines der folgenden Merkmale umfasst:
- die Außenaufnahme (13) ist ein biegsamer, nicht dehnbarer oder mit einer begrenzten Dehnungsfähigkeit dehnbarer Außenbeutel (13);
- die Außenaufnahme (13) ist eine starre oder halbstarre Schale.

5. Vorrichtung (1) zur Aufnahme und anschließenden kontrollierten Entleerung unter Druck einer biopharmazeutischen Flüssigkeit nach einem der Ansprüche 1 und 2, wobei der Außenbehälter (4) die Außenaufnahme (13), die ein biegsamer Außenbeutel (13) ist, der gegebenenfalls dehnbar ist, und ein äußeres Haltemittel (20) umfasst, das dazu geeignet ist, den Außenbeutel (13) aufzunehmen und dessen Funktion darin besteht, die Dehnungsfähigkeit des Außenbeutels (13) zu begrenzen, wenn Entleerungsgas unter Druck in die Druckkammer (16) eingepresst wird.

6. Vorrichtung (1) zur Aufnahme und anschließenden kontrollierten Entleerung unter Druck einer biopharmazeutischen Flüssigkeit nach Anspruch 5, wobei das Haltemittel (20) zwei große starre Wände (22) umfasst, die zueinander parallel und voneinander, insbesondere um einen festen Abstand, beabstandet sind, zwischen denen der Außenbeutel (13) platziert wird, der zwei entgegengesetzte (21) große Wände umfasst.

7. Vorrichtung (1) zur Aufnahme und anschließenden kontrollierten Entleerung unter Druck einer biopharmazeutischen Flüssigkeit nach Anspruch 6, die ferner das eine und/oder das andere der folgenden Merkmale umfasst:
- der freie Raum (25) am Umfang der beiden großen starren Wände (22) weist die Funktion eines Durchgangs für den Außenbeutel (13) zu seiner Platzierung zwischen den beiden großen Wänden (22) oder zu seiner Entfernung daraus auf;
- das Haltemittel (20) umfasst auch eine oder mehrere starre Feldwände (26), welche die beiden großen Wände (22) starr verbinden.

8. Vorrichtung (1) zur Aufnahme und anschließenden kontrollierten Entleerung unter Druck einer biopharmazeutischen Flüssigkeit nach einem der Ansprüche 1 bis 7, die ferner ein Mittel (31a, 31b, 31c), das dazu geeignet ist, zumindest bei der Entleerung sicherzustellen, dass die Entleerungsöffnung (8) sich in Richtung des unteren Teils des Innenbeutels (3), insbesondere des untersten Teils des Innenbeutels (3), befindet, umfasst oder dazu geeignet ist, damit verbunden zu sein;
und wobei das Mittel, das dazu geeignet ist, sicherzustellen, dass die Entleerungsöffnung (8) sich in Richtung des unteren Teils des Innenbeutels (3) befindet, wahlweise entweder ein Mittel (31a) zur Aufhängung der Vorrichtung (1) in Richtung der entgegengesetzten Seite der Entleerungsöffnung (8) oder ein Mittel (31b, 31c) zur Neigung des Haltemittels (20) ist, das der Außenbehälter (4) umfasst.

9. Vorrichtung (1) zur Aufnahme und anschließenden kontrollierten Entleerung unter Druck einer biopharmazeutischen Flüssigkeit nach einem der Ansprüche 1 bis 8, die ferner das eine und/oder das andere der folgenden Merkmale umfasst:
- das integrierte Mittel (42) zum Ablassen des Gases, das die Füllleitung (43) vor dem Füllen mit der biopharmazeutischen Flüssigkeit füllt, ein Beutel (44) zur Anfangsentleerung des Gases ist, das die Füllleitung (43) füllt, der durch eine Fluidverbindung (45) verbunden ist, die in das Füllrohr (9) in der Nähe des Innenbeutels (3) eintritt, wobei auf der eintretenden Fluidverbindung (45) eine Öffnungs/Schließvorrichtung (46) vorgesehen ist und eine Öffnungs/Schließvorrichtung (27) an dem Füllrohr (9) in der Nachbarschaft der Verbindung mit der eintretenden Fluidverbindung (45) und zwischen dieser und dem Innenbeutel (3) vorgesehen ist;
- das Füllrohr (9) und das Entleerungsrohr (11) durchqueren die Wand (14) der Außenaufnahme (13), indem sie sich auf beiden Seiten davon erstrecken, durch dichte und mittels Schweißens oder eines analogen Mittels fest angebrachte Verbindungen, die auf einem Randendabschnitt dieser Wand durch zwei dieser Wand gegenüberliegende Zonen gebildet sind, die einerseits flach aneinander liegen und andererseits das Füllrohr (9) und das Entleerungsrohr (11) zwischen einander einschließen und sich dabei daran anschmiegen;
- die Wand (5) des Innenbeutels (3) umfasst einen Randendabschnitt (29a), wo sich die Füllöffnung (7) und die Entleerungsöffnung (8) einander benachbart befinden, während das Füllrohr (9) und das Entleerungsrohr (11) sich einander benachbart befinden;
- das Füllrohr (9) und das Entleerungsrohr (11) durchqueren die Wand (5) des Innenbeutels (3), indem sie sich durch dichte und mittels Schweißens oder eines analogen Mittels fest angebrachte Verbindungen von der Seite nach außen erstrecken, die auf einem Randendabschnitt (29a) dieser Wand (5) durch zwei dieser Wand gegenüberliegende Zonen gebildet sind, die einerseits flach aneinander liegen und andererseits das Füllrohr (9) und das Entleerungsrohr (11) zwischen einander einschließen und sich dabei daran anschmiegen.

10. Vorrichtung (1) zur Aufnahme und anschließenden kontrollierten Entleerung unter Druck einer biopharmazeutischen Flüssigkeit nach einem der Ansprüche 1 bis 9, die ferner das eine und/oder das andere der folgenden Merkmale umfasst:
- das Füllrohr (9) und das Entleerungsrohr (11) durchqueren die Wand (14) des Außenbehälters (13), indem sie sich auf einem Randendabschnitt (30a) dieser Wand auf beiden Seiten davon erstrecken, und durchqueren die Wand (5) des Innenbeutels (3), indem sie sich auf einem Randendabschnitt dieser Wand von der Seite nach außen erstrecken, wobei der Randendabschnitt (30a) der Wand (14) der Außenaufnahme (13) und der Randendabschnitt (29a) des Innenbeutels (3) sich im Ganzen parallel zueinander erstrecken und sich einander benachbart befinden;
- das Füllrohr (9) und das Entleerungsrohr (11) durchqueren die Wand (14) der Außenaufnahme (13), indem sie sich auf einem Randendabschnitt dieser Wand (14) auf beiden Seiten davon erstrecken, und durchqueren die Wand (5) des Innenbeutels (3), indem sie sich auf einem Randendabschnitt (29a) dieser Wand (5) von der Seite nach außen erstrecken, wobei der Randendabschnitt (30a) der Wand (14) der Außenaufnahme (13) und der Randendabschnitt (29a) des Innenbeutels (3) einander gegenüberliegend angeordnet sind, wobei der Abschnitt (9a) des Füllrohrs (9) und der Abschnitt (11a) des Entleerungsrohrs (11), die sich zwischen dem Randendabschnitt (30a) der Wand (14) der Außenaufnahme (13) und dem Randendabschnitt (29a) des Innenbeutels (3) befinden, selbsttragend sind und den Innenbeutel (3) tragen;
- die Öffnung (17) zum Einpressen von Entleerungsgas unter Druck, mit der insbesondere ein Einpressrohr verbunden ist, das einen Einpresseingang aufweist, ist in der Wand (14) der Außenaufnahme (13) mit einer dichten und mittels Schweißens oder eines analogen Mittels fest angebrachten Verbindung aufgenommen, die auf einem Randendabschnitt dieser Wand durch zwei dieser Wand gegenüberliegende Zonen gebildet ist, die einerseits flach aneinander liegen und andererseits zwischen einander die Einpressöffnung (17) aufnehmen, insbesondere das Einpressrohr zwischen einander einschließen und sich dabei daran anschmiegen;
- die Wand (14) der Außenaufnahme (13) umfasst einen Randendabschnitt, wo sich einander benachbart das Füllrohr (9), das Entleerungsrohr (11), die Einpressöffnung (17), insbesondere das Einpressrohr, befinden;
- bis auf den Abschnitt (9a) des Füllrohrs (9) und den Abschnitt (11a) des Entleerungsrohrs (11), die sich zwischen dem Randendabschnitt (30a) der Wand (14) der Außenaufnahme (13) und dem Randendabschnitt (29a) des Innenbeutels (3) befinden, die einander gegenüberliegend angeordnet sind, ist der Innenbeutel (3) frei in der Außenaufnahme (13) gelagert.

11. Vorrichtung (1) zur Aufnahme und anschließenden kontrollierten Entleerung unter Druck einer biopharmazeutischen Flüssigkeit nach einem der Ansprüche 1 bis 10, die ferner das eine und/oder das andere der folgenden Merkmale umfasst:
- wenn das Entleerungsgas in die Druckkammer (16) eingepresst wird, werden die Wand (5) des Innenbeutels (3) und die Wand (14) der Außenaufnahme (13) der Druckkammer (16) über ihren gesamten oder im Wesentlichen ihren gesamten seitlichen Umfang voneinander beabstandet;
- der Innenbeutel (3) weist eine Wand auf, die zwei große Wandabschnitte umfasst, die einander entgegengesetzt sind, und die Außenaufnahme (13) ist ein Beutel, dessen Wand zwei große, einander entgegengesetzte Wandabschnitte umfasst, derart dass, wenn der Innenbeutel (3) und der Beutel der Außenaufnahme (13) leer sind, die entsprechenden Wände sowie die Beutel selbst flach gefaltet werden und flächig angepasst werden können;
- die Außenaufnahme (13) ist zumindest teilweise transparent, derart dass die Betrachtung des Innenbeutels (3) durch seine Wand ermöglicht wird;
- der entfaltete Innenbeutel (3) weist einen Inhalt von zwischen 8 Litern und 60 Litern, insbesondere in der Größenordnung von 10 bis 50 Litern, auf, während die entfaltete Außenaufnahme (13) einen Inhalt aufweist, der mindestens gleich demjenigen des entfalteten Innenbeutels (3) ist, genauer gesagt mindestens gleich der Größenordnung von 50 Litern für einen entfalteten Innenbeutel mit Inhalt mit der Größenordnung von 10;
- die Vorrichtung umfasst ein Mittel (34) mit Druckverlust, wie beispielsweise ein Filter, das in Fluidverbindung mit dem Entleerungsrohr (11) oder dem Entleerungsausgang (12) der Kammer (6) der biopharmazeutischen Flüssigkeit steht;
- das Entleerungsrohr (11) oder der Entleerungsausgang (12) der Kammer (6) der biopharmazeutischen Flüssigkeit weist keine Pumpe, wie beispielsweise eine peristaltische Pumpe, auf.

12. System zur Aufnahme und kontrollierten Übertragung unter Druck einer biopharmazeutischen Flüssigkeit, das umfasst:
▪ eine Vorrichtung (1) zur Aufnahme und anschließenden kontrollierten Entleerung unter Druck einer biopharmazeutischen Flüssigkeit nach einem der Ansprüche 1 bis 11, insbesondere mit einem Mittel (34) mit Druckverlust, wie beispielsweise ein Filter, das in Fluidverbindung mit dem Entleerungsrohr (11) oder dem Entleerungsausgang (12) der Kammer (6) der biopharmazeutischen Flüssigkeit steht,
▪ ein Mittel, das dazu geeignet und bestimmt ist, ein Entleerungsgas unter Druck zu liefern, das eine Leitung zum Einpressen von Entleerungsgas unter Druck aufweist, das geeignet ist, in Fluidverbindung mit der Öffnung oder dem Eingang zur Einpressung von Entleerungsgas unter Druck der Vorrichtung (1) zu stehen;
▪ und ein Mittel zur Kontrolle und Steuerung des Drucks des Entleerungsgases unter Druck in der Leitung zum Einpressen von Entleerungsgas unter Druck.

13. System zur Aufnahme und kontrollierten Übertragung unter Druck einer biopharmazeutischen Flüssigkeit nach Anspruch 12, das ferner das eine und/oder das andere der folgenden Merkmale umfasst:
- das Mittel, das dazu geeignet und bestimmt ist, ein Entleerungsgas unter Druck zu liefern, liefert das Entleerungsgas unter einem Druck von mindestens gleich 70 mbar, genauer gesagt mindestens gleich 80 mbar, genauer gesagt mindestens gleich 100 mbar, genauer gesagt mindestens gleich 200 mbar, genauer gesagt mindestens gleich 300 mbar und/oder unter einem Druck von höchstens gleich 600 mbar, insbesondere höchstens gleich 500 mbar;
- das System umfasst keine Pumpe, wie beispielsweise eine peristaltische Pumpe, die mit dem Entleerungsrohr (11) oder dem Entleerungsausgang (12) der Kammer (6) der biopharmazeutischen Flüssigkeit verbunden ist.

14. Verfahren zur Aufnahme und kontrollierten Übertragung unter Druck einer biopharmazeutischen Flüssigkeit, wobei:
▪ über ein System für die Aufnahme und die kontrollierte Übertragung unter Druck einer biopharmazeutischen Flüssigkeit nach einem der Ansprüche 12 bis 13 verfügt wird, das sich dann im von biopharmazeutischer Flüssigkeit und von Entleerungsgas unter Druck und von unter kontrolliertem Druck aufzunehmender und zu übertragender biopharmazeutischer Flüssigkeit leeren Zustand befindet,
▪ wenn das Aufnehmen der biopharmazeutischen Flüssigkeit in der Vorrichtung (1) gewünscht wird, zuerst das integrierte Mittel (42) zum Ablassen des Gases, das die Füllleitung (43) füllt, eingesetzt wird und so das Gas, das die Füllleitung (43) füllt, abgelassen wird, dann die Innenkammer (6) des Innenbeutels (3) mit biopharmazeutischer Flüssigkeit über den Füllleingang (10) gefüllt wird, dann der Fülleingang (10) in den geschlossenen Zustand gebracht wird, wobei der Entleerungsausgang (12) sich im geschlossenen Zustand befindet, und die biopharmazeutische Flüssigkeit so lange wie gewünscht in der Innenkammer (6) des Innenbeutels (3) gelassen wird;
▪ und wenn das Übertragen der biopharmazeutischen Flüssigkeit unter Druck ausgehend von der Innenkammer (6) gewünscht wird:
∘ die Leitung zum Einpressen von Entleerungsgas unter Druck und der Eingang zum Einpressen von Entleerungsgas unter Druck der Außenaufnahme (13) in Fluidverbindung gebracht werden und der Entleerungsausgang (12) in den geöffneten Zustand gebracht wird,
o dann das Entleerungsgas unter Druck in die Druckkammer (16) zwischen der Außenaufnahme (13) und dem Innenbeutel (3) eingepresst wird, derart dass der Innenbeutel (3) zusammengedrückt wird und die biopharmazeutische Flüssigkeit, die sich darin befindet, unter Druck davon entleert wird.

15. Verfahren zur Aufnahme und kontrollierten Übertragung unter Druck einer biopharmazeutischen Flüssigkeit nach Anspruch 14, das ferner das eine und/oder das andere der folgenden Merkmale umfasst:
- Bringen eines Mittels (34) mit Druckverlust, wie beispielsweise ein Filter, in Fluidverbindung mit dem Entleerungsrohr (11) oder dem Entleerungsausgang (12) der Kammer (6) der biopharmazeutischen Flüssigkeit;
- beim Entleeren, Platzieren der Entleerungsöffnung (8) in Richtung des unteren Teils des Innenbeutels (3), insbesondere des untersten Teils des Innenbeutels (3);
- Einpressen des Entleerungsgases derart, dass der Druck der biopharmazeutischen Flüssigkeit im Entleerungsausgang (12) während der gesamten Entleerung im Wesentlichen konstant ist;
- Liefern des Entleerungsgases unter einem Druck von mindestens gleich 70 mbar, genauer gesagt mindestens gleich 80 mbar, genauer gesagt mindestens gleich 100 mbar, genauer gesagt mindestens gleich 200 mbar, genauer gesagt mindestens gleich 300 mbar und/oder unter einem Druck von höchstens gleich 600 mbar, insbesondere höchstens gleich 500 mbar;
- Einsetzen des integrierten Mittels (42) zum Ablassen des Gases, das die Füllleitung (43) füllt, das einen Ausgangsentleerungsbeutel (44), eine in das Füllrohr (9) eintretende Fluidverbindung (45), eine Öffnungs/Schließvorrichtung (46) auf der eintretenden Fluidverbindung (45) und eine Öffnungs/Schließvorrichtung (46) auf dem Füllrohr (9) umfasst, und wobei zum Entleeren des Gases, das die Füllleitung (43) vor dem Füllen mit der biopharmazeutischen Flüssigkeit füllt, die Öffnungs/Schließvorrichtung (46) auf dem Füllrohr (9) geschlossen wird, dann, während die Öffnungs/Schließvorrichtung (46) auf der eintretenden Fluidverbindung (45) geöffnet ist, das Füllen mit der biopharmazeutischen Flüssigkeit begonnen wird, und, wenn die biopharmazeutische Flüssigkeit die eintretende Fluidverbindung (45) erreicht, die Öffnungs/Schließvorrichtung (46) auf der eintretenden Fluidverbindung (45) geschlossen wird und die Öffnungs/Schließvorrichtung (46) auf dem Füllrohr (9) geöffnet wird;
- die Vorrichtung (1) zur Aufnahme und anschließenden kontrollierten Entleerung unter Druck einer biopharmazeutischen Flüssigkeit eingesetzt wird, in der der Außenbehälter (4) einen biegsamen Außenbeutel (13) und ein äußeres Haltemittel (20) umfasst, wobei der Außenbeutel (13) in dem äußeren Haltemittel (20) platziert wird, um die Dehnungsfähigkeit des Außenbeutels (13) zu begrenzen, wenn das Entleerungsgas unter Druck in die Druckkammer (16) eingepresst wird.

## Claims

1. A device (1) for receiving and then emptying, under pressure monitored by a laboratory for the preparation of pharmaceutical products, a biopharmaceutical fluid in large quantities, at least equal to about 10 l, with a view to subsequent treatment such as filtration, final formulation, filling of containers with smaller capacity, of the type comprising:
▪ a flexible and sealed internal bag (3) made of plastic material, having an internal enclosure (6) able and intended to receive a quantity at least equal to about 10 1 of biopharmaceutical fluid and provided with an orifice for filling (7) with the biopharmaceutical fluid and with an orifice for emptying (8) the biopharmaceutical fluid and, associated in a sealed manner with the filling orifice (7) and with the emptying orifice (8), a filling tube (9) having an inlet for filling (10) the enclosure (6) with the biopharmaceutical fluid, able to be associated with a line for filling (43) the biopharmaceutical fluid, and an emptying tube (11) having an outlet for emptying (12) the enclosure (6) from the biopharmaceutical fluid, able to be associated with a line for emptying the biopharmaceutical fluid,
▪ an external container (4) in which the internal bag (3) is placed, a compression chamber (16) being arranged between the external container (4) and the internal bag (3) whose filling inlet (10) and emptying outlet (12) are located outside the external container (4), an orifice for injecting (17) pressurized emptying gas in the compression chamber (16) being provided on said external container (4),
▪ sealed crossing of the external container (4) by the filling tube (9) and the emptying tube (11),
▪ the deformation capacities respectively of the internal bag (3) and of the external container (4) being chosen so that, when the pressurized emptying gas is injected into the compression chamber (16), the internal bag (3) is compressed and so that the biopharmaceutical fluid therein is emptied under pressure through the emptying outlet (12),
**characterized in that**:
▪ the external container (4) comprises a sealed external receptacle (13) made of plastic material, forming an external enclosure in which the internal bag (3) is placed, arranging the compression chamber (16), and including the orifice for injecting (17) pressurized emptying gas,
▪ the filling tube (9) and the emptying tube (11) pass through the external receptacle (13) by permanently fixed connections (35), the filling inlet (10) and the emptying outlet (12) being located outside the external receptacle (13),
▪ it also comprises an integrated means for purging (42) the gas filling the filling line (43) before filling with the biopharmaceutical fluid so that this gas does not enter the internal bag (3),
▪ the external receptacle (13) and the internal bag (3) form a single-use coherent whole.

2. The device (1) for receiving and then emptying under monitored pressure a biopharmaceutical fluid according to claim 1 **characterized in that**:
▪ the internal bag (3) is able and intended to receive a quantity at least equal to about 10 1 of biopharmaceutical fluid, and the deployed external receptacle (13) has a capacity of at least 40 liters,
▪ it comprises a filling tube (9) section (9a) and an emptying tube (11) section (11a) located between the edge end section (29a) of the internal bag (3) to which they are adjacent and the edge end section (30a) of the wall (14) of the external receptacle (13) through which they pass by permanently fixed sealed connections, the edge end section (29a) of the internal bag (3) and the edge end section (30a) of the wall (14) of the external receptacle (13) being disposed facing each other and separated apart from each other,
▪ it also comprises an integrated means for purging (42) the gas filling the filling line (43) before filling with the biopharmaceutical fluid so that this gas does not enter the internal bag (3), the device (1) being specially adapted to receive and then empty under pressure monitored by a laboratory for the preparation of pharmaceutical products, a biopharmaceutical fluid in large quantities, at least equal to about 10 l, with a view to subsequent treatment such as filtration, final formulation, filling of containers with smaller capacity.

3. The device (1) for receiving and then emptying under monitored pressure a biopharmaceutical fluid according to any one of claims 1 and 2, wherein the external container (4) essentially comprises, particularly consists of, the external receptacle (13).

4. The device (1) for receiving and then emptying under monitored pressure a biopharmaceutical fluid according to claim 3, further comprising one of the following characteristics:
- the external receptacle (13) is a flexible non-expandable or expandable external bag (13) with limited expansion capacity;
- the external receptacle (13) is a rigid or semi-rigid shell.

5. The device (1) for receiving and then emptying under monitored pressure a biopharmaceutical fluid according to any one of claims 1 and 2, wherein the external container (4) comprises the external receptacle (13) being a flexible, if necessary expandable, external bag (13), and an external restraining means (20) able to receive the external bag (13) and having the function of limiting the expansion capacity of the external bag (13) when the pressurized emptying gas is injected in the compression chamber (16).

6. The device (1) for receiving and then emptying under monitored pressure a biopharmaceutical fluid according to claim 5, wherein the restraining means (20) comprises two large rigid walls (22) parallel to each other and spaced apart from each other, particularly with a fixed spacing, between which the external bag (13) comprising two large opposite walls (21) is placed.

7. The device (1) for receiving and then emptying under monitored pressure a biopharmaceutical fluid according to claim 6, further comprising either or both of the following characteristics:
- the free space (25) at the periphery of the two large rigid walls (22) serves as a passage for the external bag (13) for its placement or for its removal between the two large walls (22) ;
- the restraining means (20) also comprises one or several rigid field walls (26) rigidly connecting the two large walls (22).

8. The device (1) for receiving and then emptying under monitored pressure a biopharmaceutical fluid according to any one of claims 1 to 7, which, in addition, includes or is able to be associated with a means (31a, 31b, 31c) such that, at least during emptying, the emptying orifice (8) is located towards the lower part of the internal bag (3), particularly the lowermost part of the internal bag (3);
and optionally wherein the means such that the emptying orifice (8) is located towards the lower part of the internal bag (3) is either a means (31a) for suspending the device (1) towards the opposite of the emptying orifice (8) or a means (31b, 31c) for tilting the restraining means (20) that the external container (4) comprises.

9. The device (1) for receiving and then emptying under monitored pressure a biopharmaceutical fluid according to any one of claims 1 to 8, further comprising either or both of the following characteristics:
- the integrated means for purging (42) the gas filling the filling line (43) before filling with the biopharmaceutical fluid is an initial bag for emptying (44) the gas filling the filling line (43), associated by an adventitious fluid connection (45) to the filling tube (9), in the vicinity of the internal bag (3), an opening/closing device (46) being provided on the adventitious fluid connection (45) and an opening/closing device (27) being provided on the filling tube (9) in the vicinity of the connection with the adventitious fluid connection (45) and between the latter and the internal bag (3);
- the filling tube (9) and the emptying tube (11) pass, by extending on either side, through the wall (14) of the external receptacle (13) by permanently fixed sealed connections, by welding or the like, formed on an edge end section of this wall by two areas facing this wall, on the one hand flat against each other, on the other hand trapping therebetween by conforming to the filling tube (9) and to the emptying tube (11);
- the wall (5) of the internal bag (3) includes an edge end section (29a) where the filling orifice (7) and the emptying orifice (8) are located in the vicinity of each other, while the filling tube (9) and the emptying tube (11) are located in the vicinity of each other;
- the filling tube (9) and the emptying tube (11) pass, by extending from the side towards the outside, through the wall (5) of the internal bag (3) by permanently fixed sealed connections, by welding or the like, formed on an edge end section (29a) of this wall (5) by two areas facing this wall, on the one hand flat against each other, on the other hand trapping therebetween by conforming to the filling tube (9) and to the emptying tube (11).

10. The device (1) for receiving and then emptying under monitored pressure a biopharmaceutical fluid according to any one of claims 1 to 9, further comprising either or both of the following characteristics:
- the filling tube (9) and the emptying tube (11) pass, by extending on either side, through the wall (14) of the external receptacle (13) on an edge end section (30a) of this wall and pass, by extending from the side towards the outside, through the wall (5) of the internal bag (3) on an edge end section of this wall, the edge end section (30a) of the wall (14) of the external receptacle (13) and the edge end section (29a) of the internal bag (3) extending generally parallel to each other and being located in the vicinity of each other;
- the filling tube (9) and the emptying tube (11) pass, by extending on either side, through the wall (14) of the external receptacle (13) on an edge end section of this wall (14) and pass, by extending from the side towards the outside, through the wall (5) of the internal bag (3) on an edge end section (29a) of this wall (5), the edge end section (30a) of the wall (14) of the external receptacle (13) and the edge end section (29a) of the internal bag (3) being disposed opposite each other, the filling tube (9) section (9a) and the emptying tube (11) section (11a) located between the edge end section (30a) of the wall (14) of the external receptacle (13) and the edge end section (29a) of the internal bag (3) being self-supporting and carrying the internal bag (3);
- the orifice for injecting (17) pressurized emptying gas, particularly with which an injection tube having an injection inlet is associated, is arranged in the wall (14) of the external receptacle (13) with a permanently fixed sealed connection, by welding or the like, formed on an edge end section of this wall by two areas facing this wall, on the one hand flat against each other, on the other hand arranging therebetween the injection orifice (17), particularly trapping therebetween by conforming to the injection tube;
- the wall (14) of the external receptacle (13) includes an edge end section where the filling tube (9), the emptying tube (11), the injection orifice (17), particularly the injection tube are located in the vicinity of each other;
- apart from the filling tube (9) section (9a) and the emptying tube (11) section (11a) located between the edge end section (30a) of the wall (14) of the external receptacle (13) and the edge end section (29a) of the internal bag (3) disposed facing each other, the internal bag (3) is mounted free in the external receptacle (13).

11. The device (1) for receiving and then emptying under monitored pressure a biopharmaceutical fluid according to any one of claims 1 to 10, further comprising either or both of the following characteristics:
- when emptying gas is injected into the compression chamber (16), the wall (5) of the internal bag (3) and the wall (14) of the external receptacle (13) of the compression chamber (16) are spaced apart from each other over their entire or over substantially their entire lateral periphery;
- the internal bag (3) has a wall comprising two large wall portions opposite each other and the external receptacle (13) is a bag whose wall comprises two large wall portions opposite each other, so that when the internal bag (3) and the bag of the external receptacle (13) are empty, the respective walls, as well as the bags themselves can be folded flat and shaped as a sheet;
- the external receptacle (13) is at least partly transparent so as to allow viewing the internal bag (3) through its wall;
- the deployed internal bag (3) has a capacity comprised between 8 liters and 60 liters, particularly of about 10 to 50 liters, while the deployed external receptacle (13) has a capacity at least equal to that of the deployed internal bag (3), in particular at least equal to about 50 liters for a deployed internal bag (3) with a capacity of about 10;
- the device includes a means with pressure drop (34) such as an associated filter in fluid communication with the emptying tube (11) or the emptying outlet (12) of the enclosure (6) of the biopharmaceutical fluid;
- the emptying tube (11) or the emptying outlet (12) of the enclosure (6) of the biopharmaceutical fluid is free of a pump, such as a peristaltic pump.

12. A system for receiving and transferring under monitored pressure a biopharmaceutical fluid, comprising:
▪ a device (1) for receiving and then emptying under monitored pressure a biopharmaceutical fluid according to any one of claims 1 to 11, particularly with a means with pressure drop (34) such as an associated filter in fluid communication with the emptying tube (11) or the emptying outlet (12) of the enclosure (6) of the biopharmaceutical fluid,
▪ a means able and intended to deliver a pressurized emptying gas having a pressurized emptying gas injection line, able to be associated in fluid communication with the orifice or the inlet for injecting pressurized emptying gas from said device (1),
▪ and a means for monitoring and controlling the pressure of the pressurized emptying gas in the pressurized emptying gas injection line.

13. The system for receiving and transferring under monitored pressure a biopharmaceutical fluid according to claim 12, further comprising either or both of the following characteristics:
- the means able and intended to deliver a pressurized emptying gas delivers the emptying gas under a pressure at least equal to 70 mbar, more particularly at least equal to 80 mbar, more particularly at least equal to 100 mbar, more particularly at least equal to 200 mbar, more particularly at least equal to 300 mbar and/or under a pressure at most equal to 600 mbar, more particularly at most equal to 500 mbar;
- the system is free of a pump, such as a peristaltic pump, associated with the emptying tube (11) or the emptying outlet (12) of the enclosure (6) of the biopharmaceutical fluid.

14. A method for receiving and transferring under monitored pressure a biopharmaceutical fluid, wherein:
▪ there is a system for receiving and transferring under monitored pressure a biopharmaceutical fluid according to any one of claims 12 to 13 then in the empty state of biopharmaceutical fluid and pressurized emptying gas, and of biopharmaceutical fluid to be received and transferred under monitored pressure,
▪ when it is desired to receive biopharmaceutical fluid in the device (1), the integrated means for purging (42) the gas filling the filling line (43) is first implemented and thus the gas filling the filling line (43) is purged, then the internal enclosure (6) of the internal bag (3) is filled with biopharmaceutical fluid, via the filling inlet (10) then the filling inlet (10) is brought in the closed state, the emptying outlet (12) being in the closed state, and the biopharmaceutical fluid is left in the internal enclosure (6) of the internal bag (3) as much as desired,
▪ and, when it is desired to transfer the biopharmaceutical fluid under monitored pressure from the internal enclosure (6):
∘ the pressurized emptying gas injection line and the pressurized emptying gas injection inlet of the external receptacle (13) are associated in fluid communication and the emptying outlet (12) is brought in the open state,
∘ then the pressurized emptying gas is injected into the compression chamber (16) between the external receptacle (13) and the internal bag (3), so as to compress the internal bag (3) and to empty therefrom under pressure the biopharmaceutical fluid therein.

15. The method for receiving and transferring under monitored pressure a biopharmaceutical fluid according to claim 14, further comprising either or both of the following characteristics:
- a means with pressure drop (34) such as a filter is associated in fluid communication with the emptying tube (11) or the emptying outlet (12) of the enclosure (6) of the biopharmaceutical fluid;
- during emptying, the emptying orifice (8) is placed towards the lower part of the internal bag (3), particularly the lowermost part of the internal bag (3);
- the emptying gas is injected so that the pressure of the biopharmaceutical fluid in the emptying outlet (12) is substantially constant throughout the emptying;
- the emptying gas is delivered under a pressure at least equal to 70 mbar, more particularly at least equal to 80 mbar, more particularly at least equal to 100 mbar, more particularly at least equal to 200 mbar, more particularly at least equal to 300 mbar and/or under a pressure at most equal to 600 mbar, more particularly at most equal to 500 mbar;
- an integrated means for emptying (42) the gas filling the filling line (43) is implemented, comprising an initial emptying bag (44), an adventitious fluid connection (45) to the filling tube (9), an opening/closing device (46) on the adventitious fluid connection (45) and an opening/closing device (46) on the filling tube (9), and wherein for purging the gas filling the filling line (43), before filling with the biopharmaceutical fluid, the opening/closing device (46) on the filling tube (9) is closed, then while the opening/closing device (46) on the adventitious fluid connection (45) is opened, the filling with the biopharmaceutical fluid is started, and when the biopharmaceutical fluid reaches the adventitious fluid connection (45), the opening/closing device (46) on the adventitious fluid connection (45) is closed and the opening/closing device (46) is opened on the filling tube (9);
- a device (1) for receiving and then emptying under monitored pressure a biopharmaceutical fluid is implemented, wherein the external container (4) comprises a flexible external bag (13) and an external restraining means (20), wherein the external bag (13) is placed in the external restraining means (20) to limit the expansion capacity of the external bag (13) when the pressurized emptying gas is injected into the compression chamber (16).
